# EUROPEAN PATENT APPLICATION

(11) **EP 4 108 778 A1**
(43) Date of publication of application: **28.12.2022**
(21) Application number: 22173905.5
(22) Date of filing: 05.06.2017
(51) Int. Cl.: C12P 19/56, A23L 2/60, A23L 27/30

(54) **METHODS OF PREPARING GLYCOSYLATED STEVIOL GLYCOSIDE COMPOSITIONS**

(30) Priority: 06.06.2016 US 201662346148 P
(62) Divisional of application: 17732653.5
(71) Applicant: Tate & Lyle Solutions USA LLC, Hoffman Estates, IL 60192 (US)
(72) Inventor: FLETCHER, Joshua, Hoffman Estates, IL 60192 (US); WOODYER, Ryan, Hoffman Estates, IL 60192 (US); GOLOLOBOV, Mikhail, Hoffman Estates, IL 60192 (US); GROSS, Ryan, Hoffman Estates, IL 60192 (US); SMYTHE, John, Hoffman Estates, IL 60192 (US); SIRAJ, Salma, Hoffman Estates, IL 60192 (US); CHEN, Xian, Hoffman Estates, IL 60192 (US); GADDY, James, Hoffman Estates, IL 60192 (US)
(74) Representative: Marks & Clerk LLP

(57) **Abstract**

Processes for the preparation of glycosylated steviol glycoside compositions useful as sweeteners and flavor modifiers in food and beverage products and the like are improved by the use of basic conditions before, during and/or after an enzyme-catalyzed glycosylation of a steviol glycoside composition.

## Description

This application is a divisional application of EP17732653.5 and claims priority to United States Provisional Application No. 62/346,148, filed 6 June 2016, the entire disclosure of which is incorporated herein by reference for all purposes.

### Field of the Invention

The invention relates to improved methods for preparing glycosylated steviol glycoside compositions, the glycosylated steviol glycoside compositions thereby obtained, sweeteners and blends including such glycosylated steviol glycoside compositions, and the use of such glycosylated steviol glycosides as sweeteners and flavor modifiers in edible products such as foods and beverages.

### Background of the Related Art

In recent years, there has been considerable interest in the development of high intensity sweeteners based on natural products. Such sweeteners are capable of being substituted for sugar in food and beverage products, thereby potentially lowering the sugar and possibly also the caloric content of such products. Steviol glycosides extracted and isolated from the leaves of the *Stevia* plant have been the subject of significant research and commercial attention, as most such compounds are many times sweeter than sugar (sucrose). Older breeds of *Stevia* plants contain stevioside as a major steviol glycoside component, together with smaller amounts of rebaudioside A and other minor steviol glycosides. Although stevioside is approximately 100 to 300 times sweeter than sucrose, its quality of taste is considered to be significantly inferior to that of rebaudioside A. Consequently, new varieties of *Stevia* plants having much higher levels of rebaudioside A and ways of processing *Stevia* leaves to obtain highly pure (95%+) rebaudioside A have been developed. However, even highly purified rebaudioside A does not have a taste profile entirely comparable to that of sucrose; thus, formulating low sugar foods and beverages using purified rebaudioside A alone has remained quite challenging.

Steviol glycosides are characterized by having glucose molecules attached to a central steviol (terpenoid) moiety. It was discovered some time ago that naturally occurring steviol glycosides such as stevioside and rebaudioside A could be modified by allowing an alpha-glucosyltransferase to react on an aqueous solution containing one or more steviol glycosides and a glucose donor such as starch or cyclodextrin whereby one or more glucose units are transferred from the glucose donor to the steviol glycoside(s). The reaction products thereby obtained, which are generally referred to as "glycosylated steviol glycosides," can have modified/improved sensory characteristics as compared to the starting steviol glycoside(s). Examples of such reactions, which may be considered to involve glycosylation or transglycosylation, are described in U.S. Pat. No. 4,219,571 and U.S. Pat. Publication No. 2007/0082102.

However, further improvements in steviol glycoside glycosylation technology would be desirable. For example, the yields of glycosylated steviol glycoside(s) obtained in such reactions tend to be low. Additionally, the flavor and taste of glycosylated steviol glycosides prepared using known methods, while perhaps better than that of the starting steviol glycoside(s), may still not be completely ideal, in that they differ in noticeable ways from the sensory characteristics possessed by sucrose.

### Summary of the Invention

Various non-limiting exemplary aspects of the invention may be summarized as follows:
Aspect 1: A method of making a glycosylated steviol glycoside composition, comprising, consisting essentially of or consisting of contacting a starting steviol glycoside composition, a glucose donor and a cyclodextrin glycosyltransferase in an aqueous medium having a pH of greater than 7.5 to not more than 10 for a time effective to produce the glycosylated steviol glycoside composition.
Aspect 2: The method of Aspect 1, wherein the starting steviol glycoside composition is comprised of, consists essentially of or consists of one or more steviol glycosides selected from the group consisting of rebaudioside A, rebaudioside B, rebaudioside C, rebaudioside D, rebaudioside F, rebaudioside M (X), stevioside, steviolbioside, rubusoside, and dulcoside A.
Aspect 3: The method of Aspect 1, wherein the starting steviol glycoside composition is comprised of at least about 50% rebaudioside B on a dry weight basis.
Aspect 4: The method of Aspect 1, wherein the starting steviol glycoside composition is comprised of at least about 50% rebaudioside A on a dry weight basis.
Aspect 5: The method of Aspect 1, wherein the starting steviol glycoside composition is comprised of at least about 50% stevioside on a dry weight basis.
Aspect 6: The method of Aspect 1, wherein the starting steviol glycoside composition is comprised of at least about 10% steviolbioside on a dry weight basis.
Aspect 7: The method of any of Aspects 1 to 6, wherein the glucose donor is selected from the group consisting of oligosaccharides, cyclodextrins and polysaccharides.
Aspect 8: The method of any of Aspects 1 to 6, wherein the glucose donor is selected from the group consisting of starches, dextrins, liquefied starches and combinations thereof.
Aspect 9: The method of any of Aspects 1 to 8, wherein the pH is from about 8 to about 9.
Aspect 10: The method of any of Aspects 1 to 9, wherein the cyclodextrin glycosyltransferase is a cyclodextrin glycosyltransferase obtained or derived from *Bacillus stearothermophilus, Bacillus macerans, Bacillus circulans, Bacillus alcalophilus, Bacillus halophilus,* a *Thermoanaerobacter* strain or a *Thermoanaerobium* strain.
Aspect 11: The method of any of Aspects 1 to 9, wherein the cyclodextrin glycosyltransferase is a cyclodextrin glycosyltransferase obtained or derived from a strain of *Thermoanaerobacter sp.*
Aspect 12: The method of any of Aspects 1 to 9, wherein the cyclodextrin glycosyltransferase is a non-alkaliphilic cyclodextrin glycosyltransferase.
Aspect 13: The method of any of Aspects 1 to 12, wherein the contacting is carried out at a temperature of from about 20°C to about 95°C.
Aspect 14: The method of any of Aspects 1 to 13, wherein the contacting is carried out for a time of from about 1 hour to about 250 hours.
Aspect 15: The method of any of Aspects 1 to 14, wherein the aqueous medium is a buffered aqueous medium.
Aspect 16: The method of any of Aspects 1 to 15, comprising an additional step of contacting the glycosylated steviol glycoside composition with at least one enzyme capable of cleaving glucose-to-glucose bonds.
Aspect 17: The method of any of Aspects 1 to 16, wherein the contacting step is carried out under conditions effective to achieve from 60 to 85% or from 65% to 80% conversion of the steviol glycosides present in the starting steviol glycoside composition to glycosylated steviol glycosides.
Aspect 18: A method of making a glycosylated steviol glycoside composition, comprising, consisting essentially of or consisting of a) contacting a starting steviol glycoside composition with a basic aqueous medium or a basic resin to obtain a base-treated steviol glycoside composition and b) contacting the base-treated steviol glycoside composition, a glucose donor and a cyclodextrin glycosyltransferase in an aqueous medium for a time effective to produce the glycosylated steviol glycoside composition.
Aspect 19: The method of Aspect 18, wherein the starting steviol glycoside composition is contacted with a basic aqueous medium having a pH of from 7.5 to 14. Aspect 20: The method of Aspect 18 or 19, wherein step a) is carried out at a temperature of from 20°C to 100°C.
Aspect 21: The method of any of Aspects 18 to 20, wherein step a) is carried out for a time of from 0.5 to 250 hours or 1 to 250 hours.
Aspect 22: The method of any of Aspects 18 to 21, wherein the starting steviol glycoside composition is comprised of, consists essentially of or consists of at least one of rebaudioside A or stevioside.
Aspect 23: The method of Aspect 22, wherein step a) is carried out under conditions effective to convert at least a portion of the rebaudioside A, if present, to rebaudioside B and at least a portion of the stevioside, if present, to steviolbioside.
Aspect 24: The method of any of Aspects 18 to 23, comprising an additional step of contacting the glycosylated steviol glycoside composition with at least one enzyme capable of cleaving glucose-to-glucose bonds.
Aspect 25: A method of improving one or more sensory characteristics of a glycosylated steviol glycoside composition, comprising, consisting essentially of or consisting of contacting the glycosylated steviol glycoside composition with a basic aqueous medium or a basic resin.
Aspect 26: The method of Aspect 25, wherein the glycosylated steviol glycoside composition is contacted with a basic aqueous medium and the basic aqueous medium has a pH of from 7.5 to 14 or 8 to 14.
Aspect 27: The method of Aspect 25 or 26, wherein the contacting step is carried out at a temperature of from 20°C to 100°C.
Aspect 28: The method of any of Aspects 25 to 27, wherein the glycosylated steviol glycoside composition is comprised of, consists essentially of or consists of at least one of glycosylated rebaudioside A or glycosylated stevioside.
Aspect 29: The method of Aspect 28, wherein the contacting step is carried out under conditions effective to convert at least a portion of the glycosylated rebaudioside A, if present, to glycosylated rebaudioside B and at least a portion of the glycosylated stevioside, if present, to glycosylated steviolbioside.
Aspect 30: A glycosylated steviol glycoside composition obtained by the method of any of Aspects 1 to 29.
Aspect 31: A sweetener composition comprised of, consisting essentially of or consisting of a) a glycosylated steviol glycoside composition and b) 2 to 8 % by weight of Rebaudioside B, based on the total dry weight of a) and b).
Aspect 32: The sweetener composition of Aspect 31, wherein the glycosylated steviol glycoside composition is obtained in accordance with the method of any of Aspects 1 to 29.
Aspect 33: A sweetener composition comprised of, consisting essentially of or consisting of a) a first glycosylated steviol glycoside composition and b) a second glycosylated steviol glycoside composition obtained by the method of any of Aspects 18 to 24.
Aspect 34: The sweetener composition of Aspect 33, wherein the first glycosylated steviol glycoside composition is obtained by the method of any of Aspects 1 to 17.
Aspect 35: The sweetener composition of Aspect 33 or 34, comprised of, consisting essentially of or consisting of a) 65 to 95% by weight of the first steviol glycoside composition and b) 5 to 35% by weight of the second glycosylated steviol glycoside composition, based on the total dry weight of a) and b).
Aspect 36: A sweetener composition comprised of, consisting essentially of or consisting of a) a first glycosylated steviol glycoside composition and b) a second glycosylated steviol glycoside composition obtained by the method of any of Aspects 25-28.
Aspect 37: The sweetener composition of Aspect 36, wherein the first glycosylated steviol glycoside composition is obtained by the method of any of Aspects 1 to 17.
Aspect 38: The sweetener composition of Aspect 36 or 37, comprised of, consisting essentially of or consisting of a) 50 to 95% by weight of the first glycosylated steviol glycoside composition and b) 5 to 50% by weight of the second glycosylated steviol glycoside composition, based on the total dry weight of a) and b).
Aspect 39: A glycosylated steviol glycoside composition, wherein glycosylated rebaudioside B, glycosylated steviolbioside, steviolbioside and rebaudioside B are each present and together comprise from 5 to 50% or from 10 to 25%, on a dry weight basis, of the glycosylated steviol glycoside composition.
Aspect 40: A glycosylated steviol glycoside composition, wherein glycosylated rebaudioside B and glycosylated steviolbioside are both present and together comprise from 2 to 25% or from 5 to 15% on a dry weight basis of the glycosylated steviol glycoside composition.
Aspect 41: A food, beverage, cosmetic or pharmaceutical product comprising, consisting essentially of or consisting of i) at least one of a) a glycosylated steviol glycoside composition obtained by the method of any of Aspects 1 to 29, b) the sweetener composition of any of Aspects 31-38, or c) the glycosylated steviol glycoside composition of Aspect 39 or 40 and ii) at least one additional food, beverage, cosmetic or pharmaceutical ingredient.
Aspect 42: A method of making a food, beverage, cosmetic or pharmaceutical product comprising, consisting essentially of or consisting of combining i) at least one of a) a glycosylated steviol glycoside composition obtained by the method of any of Aspects 1 to 29, b) the sweetener composition of any of Aspects 31 to 38, or c) the glycosylated steviol glycoside composition of Aspect 39 or 40 and ii) at least one additional food, beverage, cosmetic or pharmaceutical ingredient.
Aspect 43: Use of at least one of a) a glycosylated steviol glycoside composition obtained by the method of any of Aspects 1 to 29, b) the sweetener composition of any of Aspects 31-38, or c) the glycosylated steviol glycoside composition of Aspect 39 or 40 in a food, beverage, cosmetic or pharmaceutical product.

### Description of the Drawings

Figure 1 is a process flow diagram which schematically illustrates various methods in accordance with the invention and how they may be practiced in conjunction with one another.
Figures 2-8 are LC-MS chromatograms of various steviol glycoside and glycosylated steviol glycoside compositions, as further explained in the Examples.
Figure 9 shows the format of a test sample evaluation form used in connection with obtaining certain test performance data, as described in the Examples.

### Detailed Description of Certain Embodiments of the Invention

The phrase "steviol glycoside", as used herein, refers to a steviol glycoside compound (a glycoside of steviol) found in the *Stevia* plant, such as rebaudioside A, rebaudioside B, rebaudioside C, rebaudioside D, rebaudioside E, rebaudioside F, rebaudioside M (sometimes also referred to as rebaudioside X), rubusoside, dulcoside or stevioside. In naturally occurring steviol glycosides, the glucose units on the steviol backbone are attached in the beta configuration.

The phrase "glycosylated steviol glycoside", as used herein, refers to a steviol glycoside that is glycosylated at one or more positions (e.g., one, two, three or more glucose moieties have been added to the steviol glycoside, through covalent glycoside linkages). In particular, a glycosylated steviol glycoside is a steviol glycoside in which one or more glucose moieties or alpha 1-4 linked glucose polymers (e.g., maltose, maltotriose, maltotetraose) are introduced on a parent steviol glycoside molecule by 1-4 linkages to a sugar moiety on the parent steviol glycoside. In a glycosylated steviol glycoside, the glucose unit(s) added by the enzymatic glycosylation reaction are attached in the alpha configuration. Accordingly, a glycosylated steviol glycoside has a structure that is different from that of a naturally occurring steviol glycoside.

The phrase "glycosylated steviol glycoside composition(s)", as used herein, is intended to mean a composition which includes one or more glycosylated steviol glycosides, but which may also include one or more substances other than glycosylated steviol glycosides (such as, for example, unreacted steviol glycoside(s) and/or unreacted glucose donor and/or carbohydrate products obtained from the glucose donor).

The present invention encompasses methods corresponding to at least three primary embodiments, which shall be referred to generally as Embodiment A, Embodiment B and Embodiment C and which are described in more detail below. These primary embodiments may be practiced independently or in combination with each other.

### Embodiment A

Embodiment A involves a method of making a glycosylated steviol glycoside composition, wherein the method comprises contacting a starting steviol glycoside composition, a glucose donor and a cyclodextrin glycosyltransferase in an aqueous medium having a pH of greater than 7.5 to not more than 10 for a time effective to produce the glycosylated steviol glycoside composition. Carrying out glycosylation under such conditions has been discovered to provide one or more of the following advantages, as compared to CGTase-catalyzed glycosylation at lower pH values: increased yield of glycosylated steviol glycosides, glycosylated steviol glycoside compositions having improved taste, and/or glycosylated steviol glycoside compositions having increased sweetness.

### Starting Steviol Glycoside Compositions

The starting steviol glycoside composition may comprise one or more steviol glycosides, which may be natural steviol glycosides (i.e., steviol glycosides found in nature in the leaves of a *Stevia* plant) and/or non-natural steviol glycosides (i.e., steviol glycosides not found in *Stevia* leaves or other natural sources). The starting steviol glycoside composition may be characterized as a composition having one or more attributes, in particular one or more sensory characteristics, which have been identified as needing improvement. For example, the attribute needing improvement may be selected from the group consisting of bitterness, sweet aftertaste, sweetness intensity, licorice flavor, astringency and combinations thereof. The starting steviol glycoside composition may comprise one or more substances other than steviol glycosides, in particular one or more of the non-steviol glycoside substances present together with steviol glycosides in a *Stevia* plant material, an extract obtained or prepared from a *Stevia* plant material or a synthetically prepared steviol glycoside composition. In various advantageous embodiments of the invention, however, the starting steviol glycoside composition is comprised of, in total, at least 50, at least 60, at least 70, at least 80, at least 90, at least 95, or at least 99 % by weight steviol glycoside(s), on a dry solids basis.

Illustrative steviol glycosides suitable for use in the starting steviol glycoside composition may be selected from the group consisting of stevioside, rebaudioside A, rebaudioside B, rebaudioside C, rebaudioside D, rebaudioside E, rebaudioside F, rebaudioside X (M), rebaudioside N, rebaudioside O, dulcoside A, steviolbioside, rubusoside, and combinations thereof. The starting steviol glycoside composition may be an extract of steviol glycosides from the leaves of a *Stevia* plant, including any of the known varieties (natural and hybridized) of *Stevia* plant. Such extracts are generally mixtures of different steviol glycosides, with stevioside and rebaudioside A typically being the steviol glycosides present in greatest abundance (depending upon the variety of *Stevia* plant from which the extract was obtained). In one embodiment, the starting steviol glycoside composition may comprise 10-70% stevioside and 20-70% rebaudioside A on a dry weight basis, wherein the total amount of stevioside and rebaudioside A is not less than 70% on a dry weight basis, with other steviol glycosides optionally being present to provide a total steviol glycoside content of not less than 90% or not less than 95% on a dry weight basis. For example, the starting steviol glycoside composition may be an extract comprised of (on a dry weight basis) 28-30% stevioside, 50-55% rebaudioside A, 9-12% rebaudioside C, 1-3% rebaudioside F and other steviol glycosides amounting to a total steviol glycoside content of at least 90% or at least 95%. In another embodiment, the starting steviol glycoside composition may be an extract comprised of (on a dry weight basis) 25-30% stevioside, 55-65% rebaudioside A and other steviol glycosides amounting to a total steviol glycoside content of at least 95%. In yet a further embodiment, the starting steviol glycoside composition may be an extract comprised of (on a dry weight basis) 55-65% rebaudioside A, 20-30% stevioside and 3 to 8% in total of rebaudioside C and dulcoside A, having a total steviol glycoside content of at least 90%. Alternatively, *Stevia* extracts with different ratios of steviol glycosides as well as highly purified (e.g., at least 80%, at least 85%, at least 90%, or at least 95% pure) steviol glycosides such as stevioside, rebaudioside A, rebaudioside B, rebaudioside C, rebaudioside D, rebaudioside E, rebaudioside F, rebaudioside X (M), rebaudioside N, rebaudioside O, dulcoside A, steviolbioside, or rubusoside may be employed.

For example, the starting steviol glycoside composition may be comprised of at least about 50%, at least 60%, at least 70%, at least 80%, or at least 90% rebaudioside B on a dry weight basis. In other embodiments, the starting steviol glycoside composition may be comprised of at least 50%, at least 60%, at least 70%, at least 80%, or at least 90% rebaudioside A on a dry weight basis. In still further embodiments, the starting steviol glycoside composition may be comprised of at least 50%, at least 60%, at least 70%, at least 80%, or at least 90% stevioside on a dry weight basis. According to additional embodiments, the starting steviol glycoside composition may be comprised of at least 10%, at least 20%, at least 30%, at least 40%, at least 50%, at least 60%, at least 70%, at least 80%, or at least 90% steviolbioside on a dry weight basis.

Also suitable for use as a starting steviol glycoside composition is a steviol glycoside composition that has been base-treated in accordance with Embodiment B of the present invention.

### Glucose Donor

Glucose donors suitable for use in the present invention include any of the oligomeric, polymeric and cyclic forms of glucose that are capable of undergoing reaction in the presence of a CGTase so as to, in effect, cleave off one or more glucose units that are transferred to steviol glycoside molecules present in the starting steviol glycoside composition. Suitable glucose donors include, for example and without limitation, starches (including starches from different sources such as wheat, corn, potato, tapioca and sago), liquefied starches (including starches that have undergone partial hydrolysis catalyzed, for example, by acid and/or enzymes such as amylases), dextrins (including maltodextrins), cyclodextrins and the like and combinations thereof. In one advantageous embodiment, the glucose donor is a maltodextrin having a Dextrose Equivalent (DE) value of from about 0.5 to about 10.

In one embodiment of the invention, a suitable glucose donor composition is prepared by treating starch or other suitable polysaccharide with a mixture of an amylase and a CGTase under conditions effective to liquefy the polysaccharide. For example, starch and water may be combined to form a starch suspension, to which is added an α-amylase and a CGTase. The resulting mixture may then be incubated at a suitable temperature (e.g., about 50°C to about 90°C or about 70°C to about 90°C) for a period of time (e.g., about 0.2 to about 6 hours) effective to liquefy the starch (resulting in a lowering of its molecular weight and an increase in its DE value). The amylase may then be inactivated by any suitable method, such as low pH heat treatment, to yield a glucose donor composition that is then combined with the starting steviol glycoside composition and an additional portion of CGTase and subjected to glycosylation conditions.

In various embodiments of the invention, an amount of glucose donor is used in the glycosylation reaction which is effective to provide a weight ratio of glucose donor to steviol glycoside (on a dry weight basis) of from about 0.5:1 to about 2:1. In other embodiments, the glucose donor:steviol glycoside weight ratio may be 1:1 to 2:1 or 1.5:1 to 2:1.

### CGTase

The cyclodextrin glycosyltransferase (CGTase) may be any of the enzymes known in the art which is capable of catalyzing the addition of glucose units onto steviol glycosides. Combinations of different CGTase enzymes may be utilized. The CGTase may be produced by mesophilic, thermophilic, alkaliphilic as well as halophilic bacilli. Suitable CGTase enzymes may, for example, be cultured or derived from *Bacillus* species such as *Bacillus stearothermophilus, Bacillus macerans, Bacillus circulans, Bacillus alcalophilus* and/or *Bacillus halophilus* and well as from strains of *Thermoanaerobacter* or *Thermoanaerobium.* Examples of such CGTases include, for example, those which are natural or recombinant enzymes derived from species of the aforementioned microorganisms. For example, a CGTase may be prepared by inoculating a sterilized culture medium with a suitable *Bacillus* species and then culturing the inoculated medium, preferable with aeration and agitation, at a temperature of from about 20°C to about 90°C for a period of time of from about 12 to 48 hours, filtering the obtained culture broth to separate the *Bacillus* cells, and further concentrating the cell-free permeate using, for example, ultrafiltration. Such enzymes may also be used in combination with one or more amylases to liquefy a starch to provide a glucose donor suitable for use in the glycosylation reaction. The CGTase enzyme(s) can be in the form of a cell-free culture broth, a concentrated liquid cell-free culture broth, a spray dried or freeze dried cell-free culture broth, or a high purity protein. Free as well as immobilized enzyme preparations can be used. In one embodiment of the invention, the CGTase is immobilized on a suitable support (for example, by gel entrapment, adsorption or covalent linking). A suitable CGTase may also be produced according to a procedure wherein a CGTase gene from a selected microorganism (e.g., a species or strain of *Thermoanaerobacter*) is isolated and cloned into a suitable host microorganism, such as *E. coli,* wherein the CGTase is expressed. Suitable CGTases are available from commercial sources such as, for example, Novozymes and Amano.

In one embodiment of the present invention, the CGTase enzyme is cultured or derived from a non-alkaliphilic organism (i.e., an organism which is not an alkaliphilic organism, which is understood to mean an organism that exhibits optimal growth at a pH of 9 or greater). According, the CGTase enzyme in a preferred embodiment of the invention is cultured or derived from an organism that exhibits optimal growth at a pH of less than 9. In particular embodiments, the CGTase enzyme is cultured or derived from a strain of *Thermoanaerobacter,* such as *Thermoanaerobacter sp.* ATCC53627. Accordingly, a *Thermoanaerobacter* CGTase, such as the CGTase enzyme sold under the brand name Toruzyme^{®} 3.0 L by Novozymes, is preferably employed in the glycosylation reaction of the present invention. In certain embodiments of the invention, glycosylation of a starting steviol glycoside composition is carried out using a CGTase having an amino acid sequence at least 70%, at least 75%, at least 80%, at least 85%, at least 90%, at least 95%, at least 99% or even 100% identical to the amino acid sequence of the CGTase having GenBank Accession No. Z35484 (see Joergensen et al., Cloning and nucleotide sequence of a thermostable cyclodextrin glycosyltransferase gene from Thermoanaerobacter sp. ATCC 53627 and its expression in Escherichia coli" Biotechnol. Lett. 19, 1027-1031 (1997)).

Previously published literature on the glycosylation of stevioside using *Thermoanaerobacter* CGTases such as Toruzyme^{®} 3.0 L CGTase clearly showed that the optimal pH for glycosylation is about 5.5, with a marked decrease in the percent of stevioside converted when the pH is increased to 7 (Li et al., "Transglycosylation of stevioside to improve the edulcorant quality by lower substitution using cornstarch hydrolyzate and CGTase" Food Chem. 138, 2013 2064-2069). A similar optimal pH was found by Mathew et al. as reported in "Regioselective glycosylation of hydroquinone to α-arbutin by cyclodextrin glucanotransferase from Thermoanaerobacter sp. Biochem. Eng. J. 79, 2013, 187-193", who showed that a pH of around 5.5 is optimal for multiple substrates with this enzyme. Furthermore, when used to produce cyclodextrin from starch, Toruzyme^{®} 3.0 L was found to have an optimal operating pH of 5 with a decrease in activity when pH is increased from pH 6 to 10. Thus, across a wide range of reactions inclusive of the glycosylation of steviol glycosides. it has been demonstrated that the expected optimal pH would be in the 5-6 range for a CGTase such as Toruzyme^{®} 3.0L. Accordingly, the finding by the present inventors that in fact certain benefits are achieved by carrying out a glycosylation of a steviol glycoside composition in an aqueous medium having a pH of greater than 7.5 to not more than 10 in the presence of a CGTase such as Toruzyme^{®} 3.0 L and a glucose donor was unexpected and could not reasonably have been predicted based on prior knowledge in the field. These benefits may include, but are not limited to, improvements in yield (i.e., an increased yield of glycosylated steviol glycoside within a predetermined period of time), taste quality (i.e., the glycosylated steviol glycoside composition obtained has better sensory properties) and/or extent of glycosylation (i.e., production of a glycosylated steviol glycoside composition having a higher number of glucose moieties added to the starting steviol glycoside(s)).

The amount of CGTase utilized in the glycosylation reaction may, in various embodiments of the invention, be about 0.02 to about 10 units of CGTase per gram of glucose donor (on a solids basis).

### Glycosylation Conditions

As mentioned previously, glycosylation of the starting steviol glycoside composition is carried out by contacting the starting steviol glycoside composition with the glucose donor in the presence of at least one CGTase in an aqueous medium. It has been discovered that the yield of glycosylated products obtained within 24 hours may be significantly increased by providing the aqueous medium with a somewhat basic pH, e.g., a pH of greater than 7.5 but not more than 10, a pH of from 8 to 10, a pH of from 8 to 9 or a pH of about 8.5. This result was surprising, as previously it has been conventional to conduct CGTase-catalyzed glycosylation of steviol glycosides at pH values that were slightly acidic (e.g., a pH of 5.5 to 6.5), particularly when the CGTase used is a non-alkaliphilic CGTase such as Toruzyme^{®} 3.0 L. The pH of the aqueous medium may be adjusted to the desired value using any suitable base or combination of suitable bases; suitable bases may include weak as well as strong bases. The base may be an inorganic base (e.g., an alkali metal hydroxide, carbonate or bicarbonate) or an organic base (e.g., organic amines, conjugate bases of organic acids). In one embodiment, the base or bases is or are solubilized in the aqueous medium. The aqueous medium may be buffered or unbuffered at the selected basic pH. In one embodiment of the invention, at least a portion of the base needed to impart the desired pH to the aqueous medium is supplied by means of the base present in the reaction product obtained by base-treating a starting steviol glycoside composition in accordance with Embodiment B of the present invention as described elsewhere herein. That is, a base-treated steviol glycoside composition may be utilized as the starting steviol glycoside composition in Embodiment A of the present invention.

The aqueous medium is comprised of water, although it is possible for the aqueous medium to be additionally comprised of one or more solvents other than water. Typically, such additional solvent(s), if present, would be miscible with water and present in relatively small amounts relative to the amount of water (e.g., the amount of solvent other than water may be less than 20, less than 10 or less than 5 % by weight based on the total weight of water and solvent). In certain embodiments, the aqueous medium does not contain any solvent other than water.

The temperature at which the glycosylation reaction is carried out is not believed to be critical, but typically the glycosylation is performed within the temperature range of from room temperature (e.g., 20°C) to about 95°C. In one embodiment, the glycosylation reaction temperature is from about 45°C to about 70°C. In another embodiment, the glycosylation reaction temperature is from about 45°C to about 55°C. Contacting of the reactants is carried out for a period of time effective to achieve a desired conversion of the starting steviol glycosides to glycosylated steviol glycosides. For example, in certain embodiments of the invention 60% to 85% or 65% to 80% conversion of the starting steviol glycosides is achieved; the extent of conversion may be readily monitored by HPLC and/or LC-MS analytical methods. The content of glycosylated steviol glycoside and the content of unreacted steviol glycoside in a glycosylated steviol glycoside composition (obtained as a glycosylation reaction product) may be determined by the procedures described on pages 257-258 of the 8th Edition of Japan's Specifications and Standards for Food Additives, published in 2009 by The Ministry of Health, Labour and Welfare.

Generally speaking, reaction times of from 1 to 250 hours or from 1 to 168 hours may be suitable, depending upon temperature, the activity of the CGTase, the composition of the starting steviol glycoside composition, enzyme concentration, and other factors. In one embodiment, the reaction time is from about 12 to about 24 hours. Where the starting steviol glycoside composition includes stevioside, for example, the reaction mixture obtained may be a relatively complex mixture of products which are mono-, di-, tri- and more highly glycosylated both at the 19-0-glucosyl unit and the terminal glucosyl unit of the 13-O-sophorosyl moiety.

According to certain embodiments, the glycosylation reaction is conducted under conditions effective to achieve only partial conversion of the steviol glycosides present in the starting material. For example, the glycosylation reaction may be stopped once approximately 60-85% of the starting steviol glycosides have reacted (i.e., have been glycosylated with one or more glucose moieties), leaving approximately 15-40% unreacted starting steviol glycosides in the reaction mixture (where the total amount of glycosylated steviol glycoside + unreacted steviol glycoside = 100%). According to another embodiments, glycosylation is carried out until approximately 65-80% of the starting steviol glycosides have reacted (i.e., have been glycosylated with one or more glucose moieties), leaving approximately 20-35% unreacted starting steviol glycosides in the reaction mixture (where the total amount of glycosylated steviol glycoside + unreacted steviol glycoside = 100%).

In various embodiments of the invention, the glycosylation reaction may be carried out using about 4 to about 6 weight % starting steviol glycoside composition and about 8 to about 12 weight % dextrin (the weight ratio of dextrin to starting steviol glycoside being from about 1.5:1 to about 2.5:1) in water (initial pH of about 8 to about 8.4). This mixture is incubated with CGTase for about 0.5 to about 30 hours at about 40°C to about 60°C to obtain a reaction product containing the desired glycosylated steviol glycosides. The enzyme is then deactivated and the reaction product purified using adsorbent resin, carbon filtration and ion exchange.

Once the desired extent of conversion has been attained (which may be determined using standard high performance liquid chromatography (HPLC) or liquid chromatography - mass spectrometry (LC-MS) techniques), the reaction product may be further treated or processed. For example, the CGTase may be deactivated (e.g., by heat treatment, wherein the reaction product is heated to a temperature effective to render the CGTase inactive with respect to its ability to catalyze further glycosylation). In other embodiments, the reaction product may be treated further with base in accordance with Embodiment C as described herein. In another embodiment, the glycosylated steviol glycoside composition may be treated with an additional, different type of enzyme, in particular with an amylase or other enzyme capable of cleaving glucose-to-glucose bonds (e.g., a maltogenic amylase, such as a maltogenic amylase derived from *Bacillus subtilis*). For example, an α-amylase may be combined with the glycosylation reaction product and the resulting mixture incubated for about 6 to 24 hours at about 55°C to about 95°C, followed by deactivation of the α-amylase (using, for example, heating at low pH). In another embodiment, an amylase such as a maltogenic amylase is combined with the glycosylation reaction product and the resulting mixture incubated for about 12 to about 36 hours at about 25°C to about 40°C. Such further enzymatic treatment serves to alter the compositional profile of the glycosylated steviol glycoside composition, thereby possibly leading to still additional improvements in the taste profile of the composition. The glycosylated steviol glycoside composition may also or alternatively be subjected to one or more of the further processing steps described herein (e.g., in the section entitled "Purification of Glycosylated Steviol Glycoside Compositions").

### Embodiment B

Embodiment B of the invention provides a method of making a glycosylated steviol glycoside composition, comprising a) contacting a starting steviol glycoside composition with a basic aqueous medium or basic resin to obtain a base-treated steviol glycoside composition and b) contacting the base-treated steviol glycoside composition, a glucose donor and a cyclodextrin glycosyltransferase in an aqueous medium for a time effective to produce the glycosylated steviol glycoside composition. Embodiment B may be practiced in combination with one or both of Embodiment A and Embodiment C, but alternatively may be carried out by itself or in combination with other processing steps known in the glycosylated steviol glycoside art. By base-treating a starting steviol glycoside composition in the manner described herein, the taste of the glycosylated steviol glycoside composition obtained from the steviol glycoside composition may be enhanced by the reduction of off-flavors, bitterness, lingering aftertaste and the like (thereby rendering the taste of the glycosylated steviol glycoside composition more like sucrose) and/or by increasing the sweetness intensity of the glycosylated steviol glycoside composition.

The starting steviol glycoside composition may correspond to any of the starting steviol glycoside compositions mentioned previously in connection with Embodiment A. However, in certain aspects of Embodiment B, the starting steviol glycoside composition contains at least one of rebaudioside A and stevioside or both rebaudioside A and stevioside. One or both of rebaudioside A and stevioside may be the predominant (≥50% by weight, ≥60% by weight, ≥70% by weight, ≥80% by weight, ≥90% by weight) steviol glycoside(s) present in the starting steviol glycoside composition. For example, rebaudioside A and stevioside may together comprise ≥50% by weight, ≥60% by weight, ≥70% by weight, ≥80% by weight, ≥90% by weight of the steviol glycoside present in the starting steviol glycoside composition.

The pH of the basic aqueous medium may, in various embodiments of the invention, be greater than 7.5, at least 8, or at least 9 and not greater than 14. For example, the basic aqueous medium may have a pH of from 8 to 14, from 9 to 14 or about 10 to about 14. The pH of the aqueous medium may be adjusted to the desired value using any suitable base or combination of suitable bases; suitable bases may include weak as well as strong bases. The base may be an inorganic base (e.g., an alkali metal hydroxide, carbonate or bicarbonate) or an organic base. In one embodiment, the base or bases is or are solubilized in the aqueous medium. The aqueous medium may be buffered or unbuffered at the selected basic pH.

In embodiments of the invention where the starting steviol glycoside composition is contacted with a basic resin, the basic resin may, for example, be a weakly basic or (preferably) strongly basic polymeric resin (e.g., a crosslinked polystyrene) having basic functional groups (such as primary, secondary and/or tertiary amino groups or quaternary ammonium groups) attached thereto. Crosslinked polystyrene sulfonates (e.g., polystyrenes crosslinked with divinylbenzene and sulfonated), wherein the sulfonate groups contain quaternary ammonium cations such as trimethylammonium, constitute one type of strongly basic resin. Polyethylene amine is an example of a suitable weakly basic resin. The starting steviol glycoside composition may be dissolved in an aqueous medium when contacted with the basic resin.

The temperature at which the base treatment of the starting steviol glycoside composition is carried out is not believed to be critical, but typically the treatment is performed within the temperature range of from room temperature (e.g., 20°C) to about 100°C. For example, the base treatment temperature may be from about 40°C to about 60°C. Base treatment is carried out for a period of time effective to achieve a desired conversion of the starting steviol glycosides, which is thought to possibly involve removal of one or more glucose units from individual steviol glycosides (in particular, removal of glucose units at the C-19 position) thereby converting them, at least in part, to other steviol glycosides. For example, if rebaudioside A is present in the starting steviol glycoside composition, at least a portion of the rebaudioside A may be converted to rebaudioside B and if stevioside is present, at least a portion of the stevioside may be converted to steviolbioside. Rebaudioside B and steviolbioside are reportedly less sweet than rebaudioside A and stevioside, respectively, with steviolbioside being characterized as having a particularly bad taste profile and low sweetness potency. However, the glycosylation products of these compounds (rebaudioside B and steviolbioside) have now been unexpectedly found to be sweeter than the glycosylation products obtained by glycosylation of the respective "parent" steviol glycosides (rebaudioside A and stevioside).

Generally speaking, reaction times of from 1 to 168 hours may be suitable, depending upon temperature, pH, base concentration, the identity of the base, and other factors. In certain embodiments of the invention, sodium hydroxide is used to adjust the pH of the aqueous medium to about 13 to about 14 and the mixture is heated at about 40°C to about 60°C for about 18 to about 30 hours.

The base-treated steviol glycoside composition thereby obtained is thereafter subjected to glycosylation by reacting with a suitable glucose donor in the presence of CGTase. Prior to glycosylation, the base-treated steviol glycoside composition may optionally be subjected to one or more processing or purification steps, such as neutralization/acidification, precipitation and the like. Suitable glucose donors and CGTases may be any of those described previously in connection with Embodiment A. The glycosylation conditions may also be the same as previously described. In various aspects of the invention, the pH of the aqueous medium may be greater than 7.5 (in accordance with Embodiment A). However, in other aspects, the pH of the aqueous medium is not greater than 7.5 (e.g., 5.5 to 7.5 or 5.5 to 6.5). The glycosylated steviol glycoside composition thereby obtained as a reaction product may be thereafter subjected to one or more further processing and/or purification steps, as described elsewhere herein or as may be known in the art, to provide a final product suitable for use as an ingredient in consumable compositions.

### Embodiment C

The present invention also provides a method of improving one or more sensory characteristics of a glycosylated steviol glycoside composition, comprising contacting the glycosylated steviol glycoside composition with a basic aqueous medium or a basic resin. This method may be practiced using any glycosylated steviol glycoside composition, i.e., any composition obtained by subjecting a steviol glycoside composition to a glycosylation reaction. For example, the glycosylated steviol glycoside composition may be a glycosylated steviol glycoside composition produced in accordance with Embodiment A or a glycosylated steviol glycoside composition produced by glycosylation of a base-treated steviol glycoside composition produced in accordance with Embodiment B of the invention. Alternatively, the glycosylated steviol glycoside composition to be base-treated may be prepared using any other glycosylation method known in the art. By processing in the manner described herein, the taste of the glycosylated steviol glycoside composition may be enhanced by the reduction of off-flavors, bitterness, lingering aftertaste and the like (thereby rendering the taste of the glycosylated steviol glycoside composition more like sucrose) and/or by increasing the sweetness intensity of the glycosylated steviol glycoside composition.

The pH of the basic aqueous medium may, in various embodiments of the invention, be greater than 7.5, at least 8, or at least 9, or at least 10 and not greater than 14. For example, the basic aqueous medium may have a pH of from 8 to 14 or from 8.5 to 12 or from 9 to 11 or about 10. The pH of the aqueous medium may be adjusted to the desired value using any suitable base or combination of suitable bases; suitable bases may include weak as well as strong bases. The base may be an inorganic base (e.g., an alkali metal hydroxide, carbonate or bicarbonate) or an organic base. In one embodiment, the base or bases is or are solubilized in the aqueous medium. The aqueous medium may be buffered or unbuffered at the selected basic pH.

In embodiments of the invention where the glycosylated steviol glycoside composition is contacted with a basic resin, the basic resin may, for example, be a weakly basic or strongly basic polymeric resin (e.g., a crosslinked polystyrene) having basic functional groups (such as primary, secondary and/or tertiary amino groups or quaternary ammonium groups) attached thereto. Crosslinked polystyrene sulfonates (e.g., polystyrenes crosslinked with divinylbenzene and sulfonated), wherein the sulfonate groups contain quaternary ammonium cations such as trimethylammonium, constitute one type of strongly basic resin. Polyethylene amine is an example of a suitable weakly basic resin. The glycosylated steviol glycoside composition may be dissolved in an aqueous medium when contacted with the basic resin.

The temperature at which the base treatment of the glycosylated steviol glycoside composition is carried out is not believed to be critical, but typically the treatment is performed within the temperature range of from room temperature (e.g., 20°C) to about 100°C. For example, the temperature may be from about 40°C to about 60°C or about 50°C. Base treatment is carried out for a period of time effective to achieve a desired conversion of the starting glycosylated steviol glycosides, which is thought to possibly involve the conversion of glycosylated rebaudioside A or glycosylated stevioside to glycosylated rebaudioside B or glycosylated steviolbioside respectively, and/or the shortening of the added glucose chains on the glycosylated steviol glycoside products. Generally speaking, reaction times of from 1 to 168 hours may be suitable (e.g., about 12 to about 36 hours or about 18 to about 30 hours), depending upon temperature, pH, base concentration, the identity of the base, and other factors. For example, when sodium hydroxide is used as the base and the aqueous medium has a pH of from about 9 to about 11, the reaction temperature may be from about 40°C to about 60°C and the reaction time may be from about 18 to about 30 hours.

The glycosylated steviol glycoside composition thereby obtained as a reaction product may be thereafter subjected to one or more further processing and/or purification steps, as described elsewhere herein or as may be known in the art, to provide a final product suitable for use as an ingredient in consumable compositions.

### Purification of Glycosylated Steviol Glycoside Compositions

Glycosylated steviol glycoside compositions prepared in accordance with any of the embodiments of the present invention may be further processed and/or purified prior to being incorporated or used as ingredients in food, beverage, cosmetic and pharmaceutical products, as will be described subsequently in further detail.

Suitable purification/processing techniques include, but are not limited to, enzyme deactivation, neutralization or other pH adjustment, filtration, sterilization, decolorization, desalting, fractionation via chromatography and the like, drying, concentration, precipitation, crystallization, treatment with adsorbents (e.g., polymeric adsorbents such as macroporous adsorbents and hydrophobic resins, activated carbon), treatment with ion exchange resins and the like and combinations thereof. If the glycosylated steviol glycoside composition is obtained in the form of an aqueous solution (e.g., a syrup), it may be employed as such or optionally converted into dry form, such as by spray drying. The glycosylated steviol glycoside composition may be combined with one or more other components (e.g., a sweetener or other flavor modifier) prior to or after being dried.

In certain embodiments of the invention, the glycosylated steviol glycoside composition is processed and/or purified using one or more of the aforementioned methods such that its content of steviol glycosides (including both unreacted starting steviol glycoside(s), if any, and the glycosylation products of the starting steviol glycoside(s)) is at least 50%, at least 60%, at least 70%, at least 80%, at least 90%, at least 95% or at least 99%, on a dry weight basis. The amount of glycosylated steviol glycosides produced may be quantified via measurement of the reduction in the amount of starting steviol glycosides using HPLC.

Depending upon the further processing/purification conditions employed, the glycosylated steviol glycoside composition obtained may contain some amount of residual glucose donor and/or carbohydrate products obtained from the glucose donor (such as maltodextrins, where the glucose donor was a starch). The composition may also or alternatively contain a portion of the steviol glycosides present in the starting steviol glycoside composition (e.g., unreacted steviol glycosides).

In various embodiments of the invention, the glycosylated steviol glycoside composition is processed and/or purified to provide a composition intended for or suitable for use as an ingredient in a consumable article (e.g., a food or beverage product) as a sweetener and/or flavorant. For example, such a glycosylated steviol glycoside composition may comprise 15 weight % to 40 weight % unreacted steviol glycoside and 60 weight % to 85 weight % glycosylated steviol glycoside on a dry solids basis, wherein the total weight of unreacted steviol glycoside and glycosylated steviol glycoside is at least 90%, on a dry weight basis, of the total weight of the glycosylated steviol glycoside composition. In other embodiments, a glycosylated steviol glycoside composition is provided which is comprised of 20 weight % to 35 weight % unreacted steviol glycoside and 65 weight % to 80 weight % glycosylated steviol glycoside on a dry solids basis, wherein the total weight of unreacted steviol glycoside and glycosylated steviol glycoside is at least 95%, on a dry weight basis, of the total weight of the glycosylated steviol glycoside composition.

According to certain embodiments, a glycosylated steviol glycoside composition is provided which contains glycosylated rebaudioside B, glycosylated steviolbioside, steviolbioside and rebaudioside B. These components may together comprise, for example, from 5 to 50% or from 10 to 25%, on a dry weight basis, of the glycosylated steviol glycoside composition. The balance of the glycosylated steviol glycoside composition may be predominantly or entirely comprised of one or more steviol glycosides and glycosylated steviol glycosides other than glycosylated rebaudioside B, glycosylated steviolbioside, steviolbioside and rebaudioside B (e.g., rebaudioside A, rebaudioside C, rebaudioside D, rebaudioside E, rebaudioside F, rebaudioside I, rebaudioside H, rebaudioside L, rebaudioside K, rebaudioside J, rebaudioside M (also referred to as rebaudioside X), rebaudioside N, rebaudioside O, dulcoside A, dulcoside B, rubusoside, stevioside, and glycosylated derivatives thereof). For example, the total amount of steviol glycosides and glycosylated steviol glycosides in the glycosylated steviol glycoside composition may be at least 85%, at least 90% or at least 95% of the glycosylated steviol glycoside composition, on a dry weight basis. In addition to the aforementioned glycosylated steviol glycosides and steviol glycosides, the glycosylated steviol glycoside composition may comprise a relatively minor amount of residual glucose donor and/or carbohydrate products obtained from the glucose donor (e.g., up to 5 or up to 10% in total of residual glucose donor and/or carbohydrate products obtained from the glucose donor, on a dry weight basis). Glycosylated steviol glycoside compositions as described above have been found to possess particularly favorable sensory properties.

According to still further embodiments, a glycosylated steviol glycoside composition is provided which contains glycosylated rebaudioside B and glycosylated steviolbioside. These components may together comprise, for example, from 2 to 25% or from 5 to 15%, on a dry weight basis, of the glycosylated steviol glycoside composition. The balance of the glycosylated steviol glycoside composition may be predominantly or entirely comprised of one or more steviol glycosides and glycosylated steviol glycosides other than glycosylated rebaudioside B and glycosylated steviolbioside (e.g., rebaudioside A, rebaudioside B, rebaudioside C, rebaudioside D, rebaudioside E, rebaudioside F, rebaudioside I, rebaudioside H, rebaudioside L, rebaudioside K, rebaudioside J, rebaudioside M (also referred to as rebaudioside X), rebaudioside N, rebaudioside O, dulcoside A, dulcoside B, rubusoside, steviolbioside, stevioside, and glycosylated derivatives thereof other than glycosylated rebaudioside B and glycosylated steviolbioside). For example, the total amount of steviol glycosides and glycosylated steviol glycosides in the glycosylated steviol glycoside composition may be at least 85%, at least 90% or at least 95% of the glycosylated steviol glycoside composition, on a dry weight basis. In addition to the aforementioned glycosylated steviol glycosides and steviol glycosides, the glycosylated steviol glycoside composition may comprise a relatively minor amount of residual glucose donor and/or carbohydrate products obtained from the glucose donor (e.g., up to 5 or up to 10% in total of residual glucose donor and/or carbohydrate products obtained from the glucose donor, on a dry weight basis). Glycosylated steviol glycoside compositions as described above have been found to possess particularly favorable sensory properties.

### Further Explanation of Exemplary Process Steps

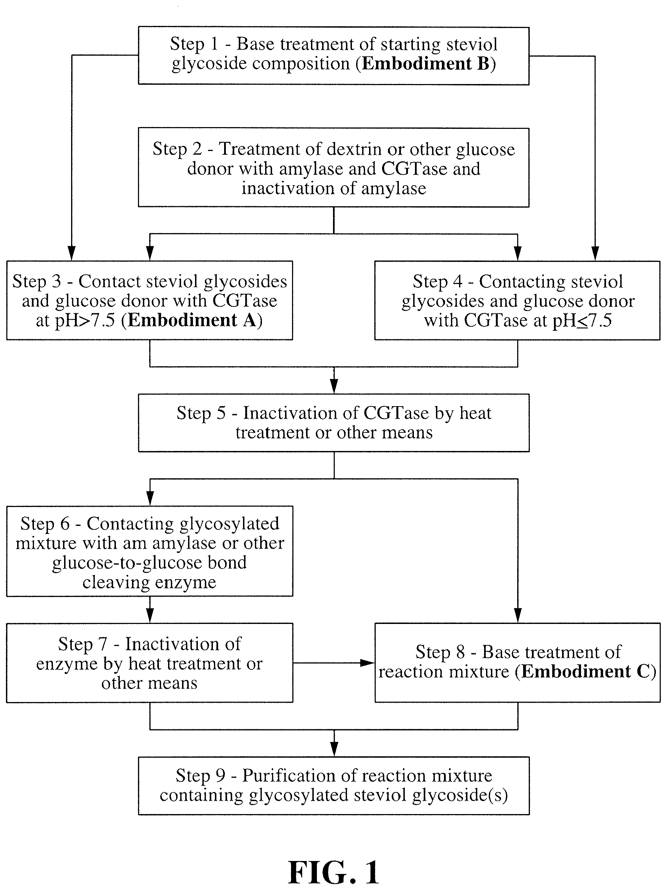

Aspects of the present invention may be further explained by reference to Figure 1, which shows in schematic form a series of processing steps which may be performed in order to convert a starting steviol glycoside composition to a glycosylated steviol glycoside composition suitable for use (for example, as a sweetener or flavorant) in a consumable product such as a food, beverage, cosmetic or pharmaceutical product. The numbering of individual steps in Figure 1 is for reference purposes only, and is not meant to imply that such steps are to be performed in numerical order or that all steps noted are necessarily performed. However, in accordance with the present invention, at least one of Step 1 (Embodiment B), Step 3 (Embodiment A) or Step 8 (Embodiment C) is carried out.

In Step 1, a starting steviol glycoside composition may optionally be subjected to a base treatment, in accordance with Embodiment B of the invention. The base-treated steviol glycoside composition may then be contacted with a glucose donor in the presence of a CGTase under conditions effective to glycosylate at least a portion of the steviol glycosides present in the base-treated steviol glycoside composition (Steps 3 and 4). Such base treatment has been found to help improve one or more sensory characteristics of the glycosylated steviol composition ultimately obtained, at least when certain types of steviol glycosides are present in the stating steviol glycoside composition. Alternatively (not shown in Figure 1), the glycosylation reaction may be conducted using a starting steviol glycoside composition that has not been treated with a base in accordance with Embodiment B. In Step 3, corresponding to Embodiment A of the invention, glycosylation is carried out using an aqueous medium having a pH greater than 7.5 (e.g., a pH of at least 8). Glycosylation under such higher pH conditions has been discovered to lead to improvements in yield, taste quality and/or extent of glycosylation. Alternatively, as shown in Step 4, conventional pH conditions (e.g., pH = 5.5-7.5) may be employed.

The glucose donor utilized in Step 3 or 4 may be an unmodified or untreated oligosaccharide or polysaccharide such as dextrin or starch. However, it may be advantageous, as shown in Step 2, to pretreat the glucose donor by subjecting it to a liquefaction step wherein it is contacted with amylase or a mixture of amylase and CGTase, followed by inactivation of the amylase (by low pH heat treatment, for example).

Once the glycosylation reaction of Step 3 or 4 has proceeded to the desired degree of conversion of the starting steviol glycoside composition (which may be monitored by HPLC or LC-MS once a specification is set, the specification being determined using organoleptic analysis of the product obtained), the CGTase may be inactivated by heat treatment or other suitable means (Step 5). The reaction product thereby obtained may then be directly subjected to purification (Step 9) to provide a glycosylated steviol glycoside composition suitable for use in consumables as described elsewhere herein. Alternatively, as illustrated in Step 8, the reaction product may be base-treated in accordance with Embodiment C of the present invention, before purification Step 9. In a still further variation (Step 6), the reaction product containing glycosylated steviol glycosides may be contacted with an amylase or other glucose-to-glucose bond cleaving enzyme under conditions effective to cause scission of at least certain glucose-to-glucose bonds that are present in the glycosylated steviol glycosides, thereby altering the types and/or relative amounts of individual glycosylated steviol glycosides present in the reaction product. The amylase or other enzyme may thereafter be inactivated (Step 7) prior to further purification of the reaction product (Step 9).

The processing steps described herein may be combined and carried out in accordance with the following exemplary process flow:
I). Base treatment of a starting steviol glycoside composition (optional if either III or VII is practiced).
II). Treatment of dextrin or other glucose donor with amylase and CGTase (optional).
III). Contacting of starting steviol glycoside composition (which may or may not be base-treated) with CGTase and glucose donor at pH >7.5 (may also be carried out at pH ≤7.5 if either I or VII is practiced).
IV). Inactivation of CGTase (by heat treatment, for example).
V). Contacting glycosylated steviol glycoside composition with an amylase or other glucose-to-glucose bond cleaving enzyme (optional).
VI). Inactivation of amylase or other enzyme (by heating, for example).
VII). Base treatment of glycosylated steviol glycoside composition (optional if either I or III is practiced).
VIII). Purification of glycosylated steviol glycoside composition (by treatment with activated carbon, ion exchange resin, and/or hydrophobic resin and/or other methods to remove impurities).

### Uses of Glycosylated Steviol Glycoside Compositions

Glycosylated steviol glycoside compositions prepared in accordance with the methods of the present invention may be used as ingredients or components of products intended for consumption, including, for example, foods, beverages, cosmetic (personal care) products and pharmaceutical products. The glycosylated steviol glycoside composition are, for example, useful as sweeteners and/or flavor modifiers or enhancers, either as the sole sweetening or flavor modifying ingredient or in combination with other sweeteners and/or flavor modifiers.

Suitable sweeteners which may be utilized in combination with the glycosylated steviol glycoside compositions prepared in accordance with the present invention include natural as well as synthetic sweeteners, nutritive and non-nutritive sweeteners, as well as high intensity and low intensity sweeteners. Sweeteners can be selected from, but are not limited to, the group consisting of sucrose, glyceraldehyde, dihydroxyacetone, erythrose, threose, erythrulose, lyxose, ribose, xylose, ribulose, xylulose, allose, altrose, galactose, glucose, gulose, idose, mannose, talose, fructose, allulose (psicose), sorbose, tagatose, mannoheptulose, sedoheptulose, octolose, fucose, rhamnose, arabinose, turanose, sialose, rebaudioside A, rebaudioside B, rebaudioside C, rebaudioside D, rebaudioside E, rebaudioside F, rebaudioside I, rebaudioside H, rebaudioside L, rebaudioside K, rebaudioside J, rebaudioside M (also referred to as rebaudioside X), rebaudioside N, rebaudioside O, dulcoside A, dulcoside B, rubusoside, *Stevia* extracts, stevioside, mogroside IV, mogroside V, Luo Han Guo extracts, siamenoside, monatin and its salts (monatin SS, RR, RS, SR), curculin, glycyrrhizic acid and its salts, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, steviolbioside and cyclocarioside I, sugar alcohols such as erythritol, sucralose, potassium acesulfame, acesulfame acid and salts thereof, aspartame, alitame, saccharin and salts thereof, neohesperidin dihydrochalcone, cyclamate, cyclamic acid and salts thereof, neotame, advantame, glucosylated steviol glycosides (GSGs) prepared by methods other than those described herein and combinations thereof.

According to an embodiment of the invention, a glycosylated steviol glycoside composition is combined with an amount of rebaudioside B effective to improve the taste and flavor characteristics of the glycosylated steviol glycoside composition. The glycosylated steviol glycoside composition may be produced in accordance with any of the procedures described herein, but may also be a glycosylated steviol glycoside composition obtained using any other method known in the art wherein a starting steviol glycoside composition is subjected to a glycosylation reaction. In certain embodiments, the glycosylated steviol glycoside composition may comprise from about 15% to about 40% by weight on a dry solids basis (e.g., from about 20% to about 35% by weight on a dry solids basis) of unreacted steviol glycosides, the balance being entirely or primarily (e.g., at least 80%, at least 85% or at least 90% by weight, on a dry solids basis) glycosylated steviol glycosides.

Combining rebaudioside B with a glycosylated steviol glycoside composition (e.g., a steviol glycoside composition obtained in accordance with the present invention) has been found to be capable of significantly improving the hedonic properties of the glycosylated steviol glycoside composition. Preferably, the glycosylated steviol glycoside composition is one that has been produced in accordance with Embodiment A (the aspect of the invention wherein a starting steviol glycoside composition, a glucose donor and a cyclodextrin glycosyltransferase are contacted in an aqueous medium having a pH of greater than 7.5 to not more than 10). In advantageous embodiments of the invention, a sweetener composition is provided which comprises, consists essentially of or consists of a glycosylated steviol glycoside composition and at least 1%, at least 2% or at least 3% by weight rebaudioside B, based on the total dry weight of the glycosylated steviol glycoside composition and rebaudioside B. The glycosylated steviol glycoside composition may contain less than 2%, less than 1% or less than 0.5% or even 0% rebaudioside B, on a dry weight basis. The sweetener composition may contain, in various embodiments, not more than 10% by weight, not more than 9% by weight, not more than 8% by weight, not more than 7% by weight, not more than 6% by weight, or not more than 5% by weight rebaudioside B, based on the total dry weight of the glycosylated steviol glycoside composition and rebaudioside B. For example, the sweetener composition may contain 1 to 10% by weight, 2 to 8% by weight, 3 to 6% by weight or about 4% by weight rebaudioside B, based on the total weight of glycosylated steviol glycoside composition and rebaudioside B.

Advantageously improved sweetener compositions may also be obtained by combining glycosylated steviol glycoside compositions made in accordance with different aspects of the present invention.

For example, the sensory performance of a glycosylated steviol glycoside composition made by contacting a starting steviol glycoside composition, a glucose donor and a cyclodextrin glycosyltransferase in an aqueous medium having a pH of greater than 7.5 to not more than 10, but no base treatment of the starting steviol glycoside composition prior to or after glycosylation ("GSG Composition Type A"), may be enhanced by combining such a composition with a glycosylated steviol glycoside composition made by a) contacting a starting steviol glycoside composition with a basic aqueous medium or a basic resin to obtain a base-treated steviol glycoside composition and b) contacting the base-treated steviol glycoside composition, a glucose donor and a cyclodextrin glycosyltransferase in an aqueous medium (having any suitable pH, e.g., about 5 to about 10; a higher yield of glycosylated steviol composition may be obtained at a somewhat basic pH, for example a pH of from about 8 to about 9) ("GSG Composition Type B"). The benefits of such blends are particularly evident at relatively high concentrations of the sweetener composition in a consumable product (e.g., at least 1000 ppm total glycosylated steviol glycoside). The amount of GSG Composition Type B in the sweetener composition may be at least 5% by weight and not more than 50% by weight (or not more than 35% by weight), based on the total dry weight of GSG Composition Type A and GSG Composition Type B. Correspondingly, the amount of GSG Composition Type A in the sweetener composition may be at least 50% by weight and not more than 95% by weight (or not more than 65% by weight), based on the total dry weight of GSG Composition Type A and GSG Composition Type B.

In another example, the sensory performance of a glycosylated steviol glycoside composition made by contacting a starting steviol glycoside composition, a glucose donor and a cyclodextrin glycosyltransferase in an aqueous medium having a pH of greater than 7.5 to not more than 10, but no base treatment of the starting steviol glycoside composition prior to or after glycosylation ("GSG Composition Type A"), may be enhanced by combining such a composition with a glycosylated steviol glycoside composition made by contacting a glycosylated steviol glycoside composition with a basic aqueous medium or a basic resin ("GSG Composition Type C"). The benefits of such blends are particularly evident at relatively high concentrations of the sweetener composition in a consumable product (e.g., at least 1000 ppm total glycosylated steviol glycoside). The amount of GSG Composition Type C in the sweetener composition may be at least 5% by weight and not more than 50% by weight, based on the total dry weight of GSG Composition Type A and GSG Composition Type C. Correspondingly, the amount of GSG Composition Type A in the sweetener composition may be at least 50% by weight and not more than 95% by weight, based on the total dry weight of GSG Composition Type A and GSG Composition Type C.

According to a further embodiment of the invention, a sweetener composition is provided which comprises, consists essentially of or consists of:
a) about 10 to about 25 (or about 15 to about 20) % by weight, on a dry solids basis, of a base-treated glycosylated steviol glycoside composition prepared in accordance with Embodiment C of the invention, wherein the glycosylated steviol glycoside composition has been obtained by glycosylation of a starting steviol glycoside composition comprised of (on a dry weight basis) 25-30% stevioside, 55-65% rebaudioside A and other steviol glycosides amounting to a total steviol glycoside content of at least 95% or a starting steviol glycoside composition comprised of (on a dry weight basis) 55-65% rebaudioside A, 20-30% stevioside and 3 to 8% in total of rebaudioside C and dulcoside A, having a total steviol glycoside content of at least 90%;
b) about 65 to about 90 (or about 70 to about 85) % by weight, on a dry solids basis, of a glycosylated steviol glycoside composition which has not been base-treated in accordance with Embodiment C of the invention, wherein the glycosylated steviol glycoside composition has been obtained by glycosylation of a starting steviol glycoside composition comprised of (on a dry weight basis) 25-30% stevioside, 55-65% rebaudioside A and other steviol glycosides amounting to a total steviol glycoside content of at least 95% or a starting steviol glycoside composition comprised of (on a dry weight basis) 55-65% rebaudioside A, 20-30% stevioside and 3 to 8% in total of rebaudioside C and dulcoside A, having a total steviol glycoside content of at least 90%; and
c) 0 to about 10 (or 0 to about 5) % by weight in total, on a dry solids basis, of residual glucose donor and/or carbohydrate products obtained from the glucose donor.

In another embodiment of the invention, a sweetener composition is provided which comprises, consists essentially of or consists of:
a) about 10 to about 25 (or about 15 to about 20) % by weight, on a dry solids basis, of a glycosylated steviol glycoside composition prepared in accordance with Embodiment A of the invention, wherein the glycosylated steviol glycoside composition has been obtained by base-treating a starting steviol glycoside composition comprised of (on a dry weight basis) 25-30% stevioside, 55-65% rebaudioside A and other steviol glycosides amounting to a total steviol glycoside content of at least 95% or a starting steviol glycoside composition comprised of (on a dry weight basis) 55-65% rebaudioside A, 20-30% stevioside and 3 to 8% in total of rebaudioside C and dulcoside A, having a total steviol glycoside content of at least 90% to obtain a base-treated starting steviol glycoside composition which is then glycosylated;
b) about 65 to about 90 (or about 70 to about 85) % by weight, on a dry solids basis, of a glycosylated steviol glycoside composition obtained by glycosylation of a starting steviol glycoside composition comprised of (on a dry weight basis) 25-30% stevioside, 55-65% rebaudioside A and other steviol glycosides amounting to a total steviol glycoside content of at least 95% or a starting steviol glycoside composition comprised of (on a dry weight basis) 55-65% rebaudioside A, 20-30% stevioside and 3 to 8% in total of rebaudioside C and dulcoside A, having a total steviol glycoside content of at least 90%, wherein the starting steviol glycoside composition has not been base-treated prior to glycosylation; and
c) 0 to about 10 (or 0 to about 5) % by weight in total, on a dry solids basis, of residual glucose donor and/or carbohydrate products obtained from the glucose donor.

One aspect of the present invention provides a food product comprising a glycosylated steviol glycoside composition prepared in accordance with the invention or a combination of such glycosylated steviol glycoside compositions, generally in combination with at least one additional food ingredient. Optionally, one or more individual steviol glycosides, including rebaudioside B in particular, may be utilized in combination with such glycosylated steviol glycoside composition(s). For example, the food product may comprise 1 to 10 weight %, 2 to 8 weight %, 3 to 6 weight % or about 4 weight % rebaudioside B in addition to such glycosylated steviol glycoside composition, based on the total dry weight of rebaudioside B and glycosylated steviol glycoside composition. The amount of glycosylated steviol glycoside composition in the food product may be varied as may be desired in order to achieve a particular desired result, such as, for example, an increase in sweetness and/or an improvement in flavor. For example, the food product may comprise a glycosylated steviol glycoside composition in a subsweetening amount or a sweetening amount. The concentration of glycosylated steviol glycoside composition in the food product, on a dry weight basis, may be from 5 to 2000 ppm. In other embodiments, the food product contains an amount of glycosylated steviol glycoside composition effective to impart an SEV of at least 7, at least 8, at least 9 or at least 10, or at least 11 to the food product, but generally not more than 20 or 15, wherein the food product may additionally contain one or more other sweeteners that also contribute to the SEV. The glycosylated steviol glycoside composition may be added into a food product also containing one or more other sweeteners in an amount effective to increase the SEV of the food product to at least 7, at least 8, at least 9, at least 10 or least 11, thereby providing a food product having a predetermined desired sweetness. Accordingly, the food product may have, in various embodiments of the invention, an SEV of 1-20, 5-15 or 7-13.

Non-limiting examples of a food product include a confectionary product (including, but not limited to, jelly candies, hard candies and gums), a dessert product such as, yogurt (including, but not limited to, full fat, reduced fat and fat-free dairy yogurts, as well non-dairy and lactose-free yogurts and frozen equivalents of all of these), frozen desserts (including, but not limited to, frozen dairy desserts such as ice-cream -including regular ice cream, soft-serve ice cream and all other types of ice cream -and frozen non-dairy desserts such as non-dairy ice cream, sorbet and the like), sweet bakery products (including, but not limited to, biscuits, cakes, rolls, pies, pastries, and cookies), pre-made sweet bakery mixes for preparing sweet bakery products, pie fillings (including, but not limited to, fruit pie fillings and nut pie fillings such as pecan pie filling), a cereal product such as sweetened breakfast cereals (including, but not limited to, extruded breakfast cereals, flaked breakfast cereals and puffed breakfast cereals), cereal coating compositions, baked goods including bread products (including, but not limited to, leavened and unleavened breads, yeasted and unyeasted breads such as soda breads, breads comprising any type of wheat flour, breads comprising any type of non-wheat flour (such as potato, rice and rye flours), gluten-free breads), pre-made bread mixes for preparing bread products, frozen dairy products, meats, dairy products, condiments, snack bars (including, but not limited to, cereal, nut, seed and/or fruit bars), soups, dressings, mixes, prepared foods, baby foods, diet preparations, syrups, food coatings, dried fruit, sauces, gravies, spreads (including, but not limited to, jams/jellies, butters and other spreadable preserves, conserves and the like). Other types of food products not mentioned here but which conventionally include one or more nutritive sweetener may also be contemplated in the context of the present invention, especially those which are reduced sugar or low sugar products. The food product may be an animal feed product, such as a pet food. The food product of the invention may comprise the sweetener composition as a coating or frosting formed on the surface of the product. This coating may improve the flavor of the food product as well as its shelf life.

The glycosylated steviol glycoside compositions of the present invention are also useful in medical foods (foods that are specially formulated and intended for the dietary management of a disease that has distinctive nutritional needs that cannot be met by normal diet alone).

Another aspect of the invention provides a beverage product comprising a glycosylated steviol glycoside composition in accordance with the present invention or a combination of glycosylated steviol glycoside compositions in accordance with the present invention, optionally modified with one or more individual steviol glycosides such as rebaudioside B. For example, the beverage product may comprise 1 to 10 weight %, 2 to 8 weight %, 3 to 6 weight % or about 4 weight % rebaudioside B in addition to such glycosylated steviol glycoside composition, based on the total dry weight of rebaudioside B and glycosylated steviol glycoside composition. The amount of glycosylated steviol glycoside composition in the beverage product may be varied as may be desired in order to achieve a particular desired result, such as, for example, an increase in sweetness and/or an improvement in flavor. For example, the beverage product may comprise a glycosylated steviol glycoside composition in a subsweetening amount or a sweetening amount. The concentration of glycosylated steviol glycoside composition (on a dry solids basis) in the beverage product may be from 5 to 2000 ppm. In one embodiment, the beverage product is comprised of 800 to 1800 ppm (dry) glycosylated steviol glycoside composition. In other embodiments, the beverage product contains an amount of glycosylated steviol glycoside composition effective to impart an SEV of at least 7, at least 8, at least 9 or at least 10, or at least 11 to the beverage product, but typically not more than 20 or 15, wherein the beverage product may additionally contain one or more other sweeteners that also contribute to the SEV. The glycosylated steviol glycoside composition may be added into a beverage product also containing one or more other sweeteners in an amount effective to increase the SEV of the beverage product to at least 7, at least 8, at least 9, at least 10 or least 11, thereby providing a beverage product having a predetermined desired sweetness. For example, a non-diet soft drink typically contains 12 grams of sucrose per 100 mL of water, i.e., 12% sucrose. Accordingly, a diet soft drink of equivalent sweetness may be formulated using an amount of glycosylated steviol glycoside composition sufficient (together with one or more other sweeteners that may be present) to achieve an SEV of 12. Accordingly, the beverage product may have, in various embodiments of the invention, an SEV of 1-20, 5-15 or 7-13.

Non-limiting examples of a beverage product include a carbonated beverage (including, but not limited to, soft carbonated beverages), a non-carbonated beverage (including, but not limited to, soft non-carbonated beverages such as flavored waters and sweet tea or coffee based beverages), a fruit-flavored beverage, a fruit juice, tea, milk, coffee especially those which are reduced sugar or low sugar products. Frozen beverage products (sometimes known as slushies) are also explicitly contemplated. Other types of beverage products not mentioned here but which conventionally include one or more nutritive sweeteners may also be contemplated in the context of the present invention, especially those which are reduced sugar or low sugar products.

A further aspect of the present invention provides a table-top sweetener comprising at least one glycosylated steviol glycoside composition in accordance with the invention. The table-top sweeteners of the present invention may optionally include one or more further ingredients selected from the group consisting of bulking agents (such as maltodextrin, polydextrose, gums such as xanthan gum or guar gum, soluble corn fiber (SCF), starches and polyols), natural and/or artificial flavors, flavor enhancers, natural and/or artificial colors, fiber, acidulants, vitamins, antioxidants, preservatives, starch hydrolyzates and the like. In one embodiment, the table-top sweetener comprises both at least one glycosylated steviol composition in accordance with the invention and at least one individual steviol glycoside such as rebaudioside B.

According to an embodiment, the table-top sweetener is a dry table-top sweetener. For example, it may take the form of tablets, granules or a powder. Liquid table-top sweeteners may also be contemplated, and typically take the form of an aqueous solution of the components.

According to an embodiment, the table-top sweetener may further comprise one or more nutritive sweeteners. The nutritive sweetener may be selected from the group consisting of sucrose, glucose, glucose syrup, isoglucose, fructose, glucose-fructose syrup, maltose, lactose, corn syrup, high fructose corn syrup, invert sugar, molasses, honey and agave. The nutritive sweetener is sucrose in one preferred embodiment. Where the table-top product includes a nutritive sweetener, said nutritive sweetener may be present in an amount of up to about 30% by weight based on the total weight of the table-top sweetener. For example, the nutritive sweetener may be present in an amount of about 26% by weight based on the total weight of the table-top sweetener. According to an embodiment, the table-top sweetener may further comprise one or more co-sweeteners selected from the group consisting of high intensity sweeteners and sugar alcohols. Various synthetic high potency sweeteners may also be used as the one or more co-sweetener. Specific examples include sucralose, aspartame and acesulfame potassium (Ace K). Various sugar alcohols may also be used as the one or more co-sweetener of the present invention. Specific examples include maltitol, xylitol and erythritol.

Table-top sweeteners according to the present invention may typically be used to sweeten beverages, especially hot beverages such as tea and coffee.

Another aspect of the present invention provides a bulking agent comprising a glycosylated steviol glycoside composition in accordance with the invention.

A further aspect of the present invention provides a coating agent comprising a glycosylated steviol glycoside composition in accordance with the invention.

A separate aspect of the present invention provides a pharmaceutical product comprising a glycosylated steviol glycoside composition in accordance with the invention and at least one other pharmaceutical ingredient such as an active ingredient or excipient. Exemplary pharmaceutical products include cough syrups, chewable tablets, lozenges, vitamin preparations and the like.

Another aspect of the present invention provides a nutritional or sports product comprising a glycosylated steviol glycoside composition in accordance with the invention.

Another aspect of the present invention provides a cosmetic (personal care) product comprising a glycosylated steviol glycoside composition in accordance with the invention and at least one cosmetic ingredient. Exemplary cosmetic products include toothpaste, mouthwash and the like.

It will be appreciated that the amount of a glycosylated steviol glycoside composition obtained in accordance with the invention which is present in a food product, a beverage product, a pharmaceutical product, a nutritional product, a sports product, or a cosmetic product, will depend upon the sweetness, taste, flavor and other sensory attributes of the glycosylated steviol glycoside composition and the type(s) and amount(s) of other sweetener(s) and/or flavor modifier(s) present in the product and the desired sweetness or other flavor characteristics of the product, as well as other factors such as the desired caloric content of the product. The glycosylated steviol glycoside composition may be used as a flavor enhancer at a concentration in the product below its sweetness detection limit (the minimum concentration at which the glycosylated steviol glycoside imparts a perceptible sweet taste to the product, in the absence of any other sweetener) or as a sweetener at a concentration in the product at or above its sweetness detection limit.

An alternative aspect of the present invention provides the use of the sweetener composition of the invention in a food product, a beverage product, a pharmaceutical product, a nutritional product, a sports product, or a cosmetic product, as a bulking agent or as a coating agent.

A glycosylated steviol glycoside composition prepared in accordance with the present invention may be formulated in any ingestible form, for example, as a syrup, in powder form, tablet form, as granules, in a solution or in any other suitable form including beverages and food products.

### Examples

### Example 1

In this Example, a high purity combination of steviol glycosides (including rebaudioside A as the predominant component) was used as a starting material and was first glycosylated. The resulting glycosylated steviol glycoside composition was then treated with base.

### Materials

1) Maltodextrin (Dextrose Equivalent = 1).
2) Cyclodextrin glucotransferase (CGTase).
3) Maltogenic amylase.
4) SG95 steviol glycoside mixture sourced from GLG Life Tech Corporation (a high purity combination of nine sweet steviol glycosides found within *Stevia* leaves, with Reb A accounting for about 60 wt% of the mixture and stevioside accounting for about 30 wt% of the mixture)
5) Granular Activated Carbon, Regenerated.
6) Strong base anionic resin and strong acid cationic resin, mixed in a 60:40 strong base anionic resin to strong acid cationic resin ratio by weight.
7) Macroporous adsorbent resin (a non-ionic aliphatic acrylic polymer having a macroreticular structure and high surface area, in bead form).

### Reaction

The procedure was performed in 500 mL fermenters. Three samples (see Table 1) of each reaction were prepared in parallel using the following procedure:
1) Suspend 20 g of SG95 steviol glycosides in 340 mL of hot water in a 1000mL beaker.
2) Add 40 g of maltodextrin (DE = 1) to the suspension and blend to submerge material.
3) Adjust pH to 8.2 using sodium hydroxide.
4) Add 2 mL of the CGTase enzyme to the solution.
5) Incubate for 24 hours at 50°C while stirring at 200 rpm.
6) Transfer to a flask and heat reaction mixture for 30 min in boiling water (approx. at 95°C) to denature the CGTase enzyme, then let cool to room temperature.
7) Return the mixture back to the fermenter beaker, rinse the flask with DI water to guarantee complete transfer of the material from the flask to the beaker and to have total volume of the mixture at approx. 500 ml.
8) Add 5 mL (1%) of the maltogenic amylase to the solution.
9) Incubate for 24 hours at 30°C while stirring at 150 rpm.
10)Heat reaction mixture for 30 min in boiling water (approx. at 95°C) to inactivate the maltogenic amylase, then let cool to room temperature.
11)Filter solution on a Buchner funnel, to remove unreacted solids/dextrins.
12)Rinse activated carbon with DI water to remove fine particles. Pack 1 #15 x 300mm jacketed column with granular activated carbon. Preheat carbon column to 50°C and continue to rinse with 1.5L of DI water to remove fine particles.
13)Prepare mixed bed resin by rinsing 29.95 g of strong base anionic resin and 20.00 g of strong acid cationic resin with DI water. Pack 1 #15 x 300mm jacketed column with mixed bed resin. Preheat column to 25°C and continue to rinse with 1.5L of DI water.
14)Filter reaction solution through the carbon and mixed bed resin columns in sequence at a flow rate of 1.5 mL/min. Rinse column with water.
15)Rinse macroporous adsorbent column with 1500 g degassed DI water.
16)Filter reaction solution through macroporous adsorbent column.
17)Rinse macroporous adsorbent column with 1500 g degassed DI water.
18)Rinse macroporous adsorbent column with 1500 g of a 50% ethanol solution (758.68 g degassed DI water and 750.63 g of 190 proof ethanol).
19)Collect ethanol rinsed solution, and evaporate the ethanol from the solution.
20)Transfer solution (740.11 g) to a 2L glass beaker. Measure pH (4.24), and adjust to a final pH of 10.03 with sodium hydroxide. Heat solution to 50°C and stir at 350 rpm for 24 hours.
21)Cool solution, and adjust pH from 8.20 to 5.00 with 7% w/w hydrochloric acid.
22)Place solution on the freeze dryer.
23)Collect dried material (24.17 g total) and submit for analytical testing.

**Table 1. Actual weights used.**

| Sample | SG95 | Water | Maltodextrin | Initial pH | Final pH |
|---|---|---|---|---|---|
| Sample 1 | 20.04 | 340.03 | 40.24 | 5.49 | 8.17 |
| Sample 2 | 20.13 | 340.02 | 40.33 | 5.54 | 8.19 |
| Sample 3 | 19.99 | 340.00 | 40.08 | 5.51 | 8.17 |

### Results

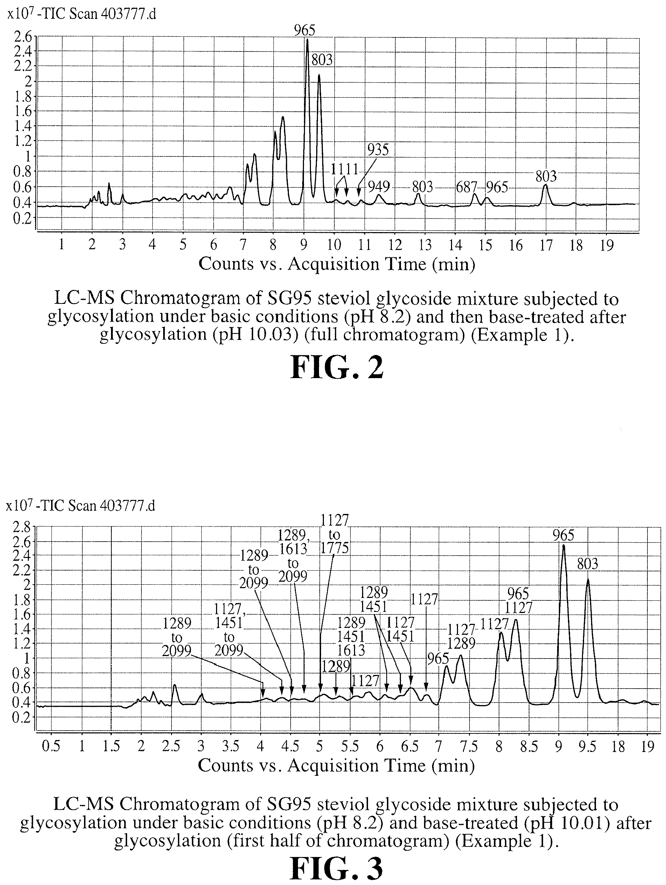

The primary analytical technique used on these samples was LC-MS. The mass spectrometer (MS) reports the molecular weight (divided by charge, which was usually 1) of the peaks from the chromatography (LC). Steviol glycosides are expected to have molecular weights as shown in the table below. The most common sugar residues added to the steviol core are glucose, rhamnose, xylose, 6-deoxyglucose, and fructose. From a mass perspective glucose = fructose, and rhamnose = 6-deoxyglucose. Another phenomenon of mass spectrometry is that adducts can form during ionization. These reaction products add mass relative to the expected mass to charge ratio. For this experiment *m*/*z* 687 seen in Figure 2 corresponds to rubusoside with a formic acid adduct. This *m*/*z* peak is also seen in a steviol glycoside standard containing rubusoside. Figure 2 shows that the majority of the reaction products correspond to mono-, di- and tri-glycosylated steviol glycosides, with the tallest peaks at 965 and 803 being rebaudioside A and stevioside, respectively.

**Table 2. Reference for mass spectroscopy data.**

| *Compound* | *m*/*z* | *Typical steviol glycosides of this mass* | *m*/*z with an added rhamnose* | *m*/*z with an added xylose* |
|---|---|---|---|---|
| Steviol | 317 | Steviol | 449 | 463 |
| 1 glc | 479 | Steviolmonoside | 611 | 625 |
| 2 glc | 641 | Steviolbioside, | 773 | 787 |
| | | Rubusoside | | |
| 3 glc | 803 | Stevioside, Rebaudioside B, Rebaudioside G | 935 | 949 |
| 4 glc | 965 | Rebaudioside A, | 1097 | 1111 |
| | | Rebaudioside E | | |
| 5 glc | 1127 | Rebaudioside D, | | |
| | | Rebaudioside I, | 1259 | 1273 |
| | | Rebaudioside L | | |
| 6 glc | 1289 | Rebaudioside M | 1421 | 1435 |
| 7 glc | 1451 | | 1583 | 1597 |
| 8 glc | 1613 | | 1745 | 1759 |
| 9 glc | 1775 | | 1907 | 1921 |
| 10 glc | 1937 | | 2069 | 2083 |
| 11 glc | 2099 | | 2231 | 2245 |
| 12 glc | 2261 | | 2393 | 2407 |

As is seen in Table 2 above, stevioside has a *m*/*z* of 803 and rebaudioside A has an *m*/*z* of 965. In Figures 2 and 3, these correspond to the large peaks which elute at retention times between 9 and 12 mins.

**Table 3. Progress of reaction under inventive and conventional conditions.**

| | ***Total Glycosides (as is peak area*)** | ***Reb B (as is peak area*)** | ***Estimated* % *reaction* (*1-reb B*/*total glycosides*)** |
|---|---|---|---|
| Inventive Conditions (higher pH) | 172469.5 | 78262.5 | **54.6%** |
| Conventional Conditions (lower pH) | 639982 | 455022 | **28.9%** |

### Discussion

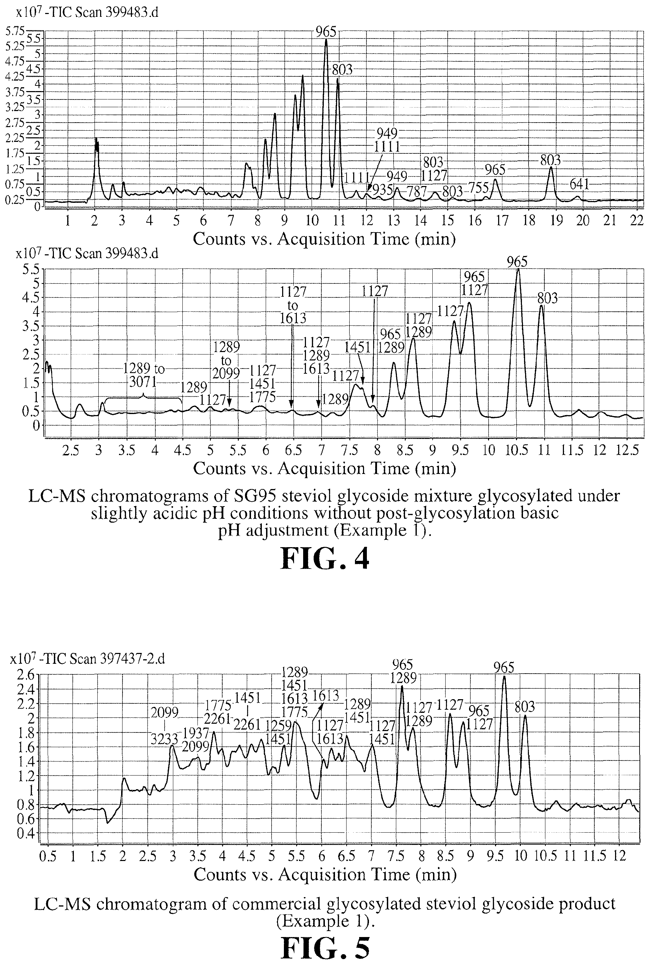

The expected major products of this procedure were glycosylated rebaudioside B and glycosylated steviolbioside, since rebaudioside B and steviolbioside are the major products of base treatment of the starting material. These products would be expected to appear in the LC-MS after 10 minutes. In general, higher degrees of glycosylation correlate with lower retention times, so products with large peaks before 7 min would be expected to contain significant quantities of steviol glycosides with a larger degree of glycosylation. While some of these products are produced, these appear to be no more prevalent than they are in the more typically prepared sample (Figure 4). The most striking difference between these figures is the suppression of the 1127 and 1451 *m*/*z* peaks at around 7.5 minutes when Figure 3 is compared to Figure 4. These peaks likely correspond to di- and tri-glycosylated rebaudioside A and tri-glycosylated stevioside. Both procedures appear to produce a lower abundance of components having high degrees of glycosylation relative to a commercial glycosylated steviol glycoside product (Figure 5).

### Example 2

This example demonstrates initial base treatment of a steviol glycoside composition, followed by transglycosylation to produce a glycosylated steviol glycoside composition.

### Base treatment

1) 39.99 g of a steviol glycoside mixture (SG95, GLG Life Tech Corporation) was dissolved in 360.00 g of a 1.25 N solution of sodium hydroxide. The pH of the resulting mixture was 13.6.
2) This mixture was heated to 50°C and stirred at 360 rpm for 24 hrs.
3) The mixture was then cooled in an ice bath.
4) Once cooled, the mixture was then brought to pH 5 (initial pH 13.60, final pH 4.99) with 7% w/w hydrochloric acid.
5) The resulting material was centrifuged three times (at 4500 rpm for 30 min at 25°C in 50 mL tubes) and dried. The precipitate was analyzed for steviol glycoside content and used in the transglycosylation step.

### Transglycosylation

1) 20 g of base-treated SG95 steviol glycoside mixture was suspended in 340 mL of hot water in a 1000mL beaker
2) 40 g of maltodextrin (DE = 1) was added to the suspension and blended to submerge the material.
3) The pH was adjusted to 8.2 and the mixture was held at 50°C.
4) 2 mL of the CGTase enzyme was added to the solution.
5) The mixture was incubated for 24 hours at 50°C while being stirred at 200 rpm.
6) The reaction mixture was then transferred to a flask and heated for 30 min in near boiling water (approx. at 95°C); the sample was then allowed to cool to room temperature.
7) The mixture was returned to the fermenter, and the flask was rinsed with DI water to guarantee complete transfer of the material from the flask to the beaker. The total volume of the mixture was approximately 500 ml.
8) 5 mL (1%) of maltogenic amylase was added to the solution.
9) The mixture was then incubated for 24 hours at 30°C while stirring at 150 rpm.
10)The reaction mixture was then transferred to a flask and heated for 30 min in near boiling water (approx. at 95°C), then the sample was allowed to cool to room temperature.
11)The mixture was then filtered through filter paper to remove poorly dispersed dextrins.
12)Granular activated carbon was then rinsed with DI water and packed into 15 x 300mm jacketed column. The carbon column was then preheated to 50°C and rinsed with 1.5L of DI water to remove fine particles.
13)The filtrate from step 11 was then passed through the carbon column at a flow rate of 1.5 mL/min. The column was thereafter rinsed with water.
14)24.13 g of strong base anionic resin was rinsed along with 16.10 g of strong acid cationic resin with DI water, and packed into a 15 x 300mm jacketed column. The mixed bed column was then heated to 25°C and rinsed with 1.5L of DI water.
15)The filtrate from step 13 was then passed through the mixed bed column at a flow rate of 1.5 mL/min. The column was thereafter rinsed with water.
16)A macroporous adsorbent column was rinsed with 1500 g degassed DI water.
17)The filtrate from step 15 was then passed through the macroporous adsorbent column, followed by 1500 g degassed DI water.
18)The macroporous adsorbent column was then rinsed with 1500 g of a 50% ethanol solution (760.69 g degassed DI water and 729.07 g of 190 proof ethanol).
19)The ethanolic fraction was collected, and dried via rotovap and freeze dryer.
20)The dried material (20.07g) was then submitted for analytical testing for heavy metals, residual solvents, and glycoside analysis.

### Results

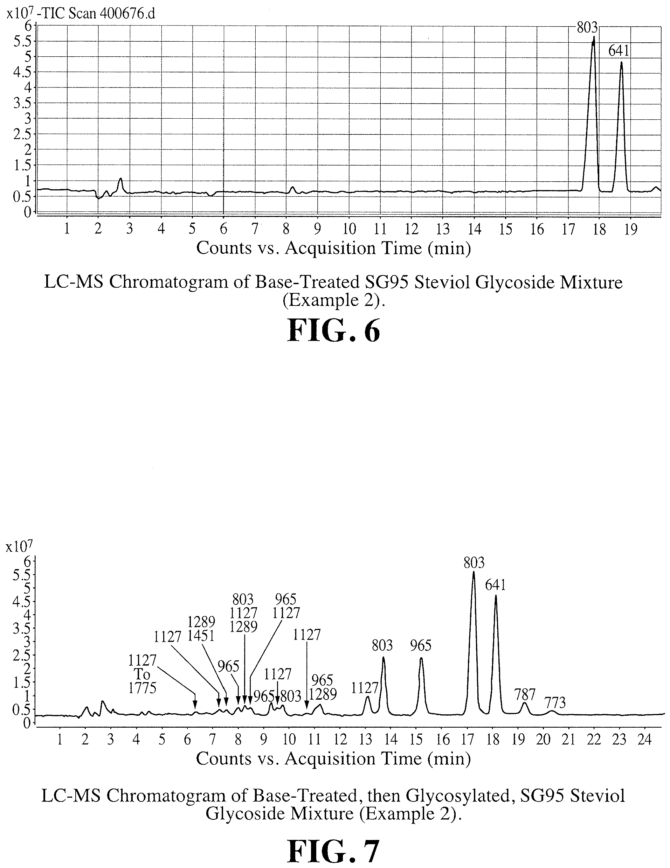

LC-MS analysis of the base-treated SG95 steviol glycoside mixture prior to enzyme treatment shows peaks corresponding to rebaudioside B and steviolbioside (Figure 6).

The LC-MS chromatogram of the enzyme-treated material is shown in Figure 7. All assigned masses correspond to sugar (glucose) additions to the steviol core.

Steviol glycosides are expected to have molecular weights as shown below in Table 4 (the designation "X glc" refers to the number of glucose units added to a steviol core):

**Table 4. Reference for mass spectroscopy data.**

| *Compound* | *m*/*z* | *Typical steviol glycosides of this mass* |
|---|---|---|
| Steviol | 317 | Steviol |
| 1 glc | 479 | Steviolmonoside |
| 2 glc | 641 | Steviolbioside, Rubusoside |
| 3 glc | 803 | Stevioside, Rebaudioside B, |
| | | Rebaudioside G |
| 4 glc | 965 | Rebaudioside A, Rebaudioside E |
| 5 glc | 1127 | Rebaudioside D, Rebaudioside I, |
| | | Rebaudioside L |
| 6 glc | 1289 | Rebaudioside M |
| 7 glc | 1451 | |
| 8 glc | 1613 | |
| 9 glc | 1775 | |
| 10 glc | 1937 | |
| 11 glc | 2099 | |
| 12 glc | 2261 | |

The large 641 and 803 peaks eluting after 17 mins are rebaudioside B, and steviolbioside. Peaks after these with *m*/*z* of 787 and 773 correspond to dulcoside B and a de-glycosylated rebaudioside F; these would be expected products of rebaudioside C and rebaudioside F base treatment. Rhamnose and xylose residues on these products likely inhibit their glycosylation.

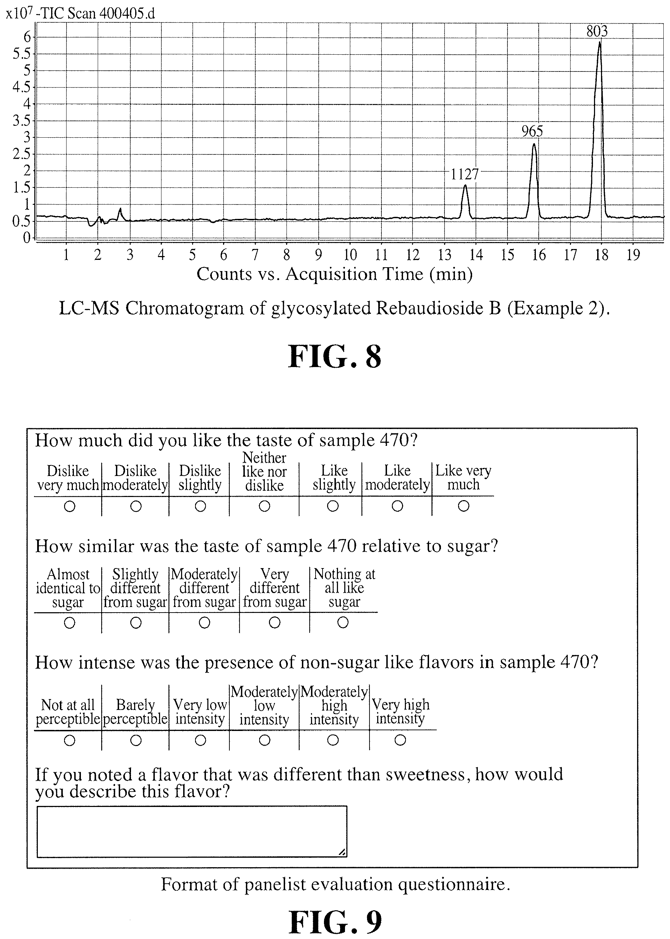

Base treatment of the SG95 steviol glycoside mixture appears to yield steviolbioside and rebaudioside B as expected. Under glycosylation conditions using an elevated (basic) pH with a 2:1 glucose donor to steviol glycoside ratio (w/w), it was predicted that the majority of the rebaudioside B (and likely the steviolbioside as well) would remain unreacted. The small peaks from 6 to 11 mins in Figure 7 are somewhat unexpected compared to previous results obtained with rebaudioside B (Figure 8), wherein glycosylation was carried out at a slightly acidic pH and a 1:1 (w/w) glucose donor: steviol glycoside ratio. For instance, the chromatogram in Figure 7 shows 4 peaks which have *m*/*z* of 803; these can only be stevioside, mono-glycosylated steviolbioside, and rebaudioside B. This implies that either steviolbioside can be glycosylated at multiple positions, or that fragmentation peaks are being recorded in the chromatogram. The relative complexity of Figure 7 relative to Figure 8, however, strongly implies that steviolbioside glycosylation is complex relative to rebaudioside B.

The structural difference between steviolbioside and rebaudioside B is that rebaudioside B has a 1-2, 1-3 linked glucose moiety, while steviolbioside only has the 1-2 linked moiety. It is possible that the reduction in steric hindrance allows both glucose residues of steviolbioside to be glycosylated, while strongly disfavoring this possibility for rebaudioside B.

### Example 3

The taste performance of various steviol glycoside compositions and glycosylated steviol glycoside compositions was evaluated. The compositions evaluated are described as follows:
Reb A = Rebaudioside A, 97% pure.
Reb A G = Glycosylated rebaudioside A (glycosylated at pH 5.5-6.0, dextrin: steviol glycoside weight ratio = 1:1).
Reb B G = Glycosylated rebaudioside B (glycosylated at pH 5.5-6.0, dextrin: steviol glycoside weight ratio = 1:1).
SG95 B-G = Glycosylated SG95 steviol glycoside mixture (sourced from GLG Life Tech Corporation, containing about 60 wt% rebaudioside A and about 30 wt% stevioside), base-treated prior to glycosylation (glycosylated at pH ca. 8.5, dextrin:steviol glycoside weight ratio = 2:1).
SG95 G-B = Glycosylated SG95 steviol glycoside mixture (sourced from GLG Life Tech Corporation, containing about 60 wt% rebaudioside A and about 30 wt% stevioside), base-treated after glycosylation (glycosylated at pH ca. 8.5, dextrin:steviol glycoside weight ratio = 2:1).
Stevioside G = Glycosylated stevioside (glycosylated at pH 5.5-6.0, dextrin: steviol glycoside weight ratio = 1:1).
Steviten = Commercial glycosylated steviol glycoside product, sold under the trade name "Steviten" by Daepyung Co., Ltd.

The Reb A G, Reb B G, SG95 B-G, SG95 G-B and Stevioside G compositions were prepared by contacting the starting steviol glycoside composition and dextrin with CGTase for 24 hours at 50°C. After a heat kill (enzyme deactivation) step, each of the compositions obtained were chain-shortened by treating with amylase for 24 hours at 30°C. After a second enzyme kill step, the compositions were purified by ion exchange resin, carbon filtration and adsorbent resin. All of the compositions were then dried using lyophilization.

Each stevia product was tested at two levels on only one day of testing, testing at use rates of both 500 ppm and 1000 ppm. Due to known carryover effects, the products were served in ascending concentration to allow for cleaner reads on both data points. The solutions were served in 2 ounce soufflé cups coded with 3-digit codes at room temperature. RO water and unsalted crackers were available for the panelists to clear their palates before and during testing. Each daily study targeted 30 persons, though at times as few as 28 panelists were tested due to exclusionary requirements (i.e., those who are lactating, pregnant, on medication, etc., who begin the study but then drop out in process).

The study was designed as a rating study using an anchored scale of references of sucrose solutions in neutral pH water. In this study, panelists received a series of reference samples labeled and identified with their SEV equivalence. The range exceeded the expected sweetness of the test sample and panelists rated the sweetness using a 15-pt maximum that exceeds the expected sweetness of the test sample.

Prescreening of the test solutions suggested most individuals would perceive these solutions as 10 SEV or less. Because one cannot predict sensitivities of all individuals effectively prior to testing, a broader range of references was used. Specifically, SEV references were as follows (Table 5):

**Table 5.**

| **2.5 SEV Reference** | **5.0 SEV Reference** | **7.5 SEV Reference** | **10.0 SEV Reference** | **12.5 SEV Reference** |
|---|---|---|---|---|
| 2.5% sucrose solution | 5.0% sucrose solution | 7.5% sucrose solution | 10.0% sucrose solution | 12.5% sucrose solution |

Panelists first were asked to familiarize themselves with the intensity of each reference solution and its corresponding SEV. Next, they waited 30sec and cleansed their palate with water and a cracker. Then panelists were ask to taste the test sample (identified by a random 3-digit code). Then they were instructed to drag a marker on a line scale to match the sweetness of their test samples relative to the references noted above, with the scale ranging from 0 to 15. A box to the right of the line scale showed the panelists the values they were dragging to so as to ensure they were not confused about the sweetness equivalence they are rating. Once this was complete, inquiries related to the liking of the test sample, the similarity of the taste of the test sample to sugar, the intensity of any off flavors, and the identity of any off flavors were asked using the format shown in Figure 9.

The values used for the liking scale were standard 7-pt hedonic scale ranging from 1 to 7, the similarity scale was a standard 5-pt intensity scale ranging from 1 to 5, and the off flavor scale was a zero-pt anchored 6-pt intensity scale ranging from 0 to 5. The description of the off flavor was open ended to allow panelists to give any names they would like to potential off notes.

The taste performance data obtained are summarized in the following table (Table 6). SEV is the sucrose equivalence value (% sucrose solution). The phrase "sucrose equivalence value" or "SEV" is the amount of non-sucrose sweetener required to provide the sweetness of a given percentage of sucrose in the same food, beverage or solution. Inversion % is the percentage of panelists who scored the 500 ppm sample as sweeter than the 1000 ppm sample. This is indicative of being near the plateau of the potency curve and of bitterness or other off-tastes masking sweetness at high concentrations. A low value of inversion % is optimal, along with a high SEV and a high liking value. Low difference from sugar scores, low SEV standard deviations, and low off-flavor scores are also desirable.

**Table 6.**

| Composition | Cone., ppm | Inversion % | SEV | SEV Std. Dev. | Liking | Diff. from Sucrose | Off-Flavor |
|---|---|---|---|---|---|---|---|
| Reb A | 500 | 28.6 | 7.2 | 2.9 | 3.6 | 2.8 | 2.6 |
| Reb A | 1000 | 28.6 | 8 | 3.1 | 2.4 | 3.6 | 3.6 |
| SG95 G-B | 500 | 16.7 | 6.3 | 2.6 | 3.7 | 2.6 | 2.4 |
| SG95 G-B | 1000 | 16.7 | 7.9 | 2.6 | 3.2 | 2.8 | 2.6 |
| Reb B G | 500 | 13.3 | 5.0 | 1.8 | 4.4 | 2.4 | 1.5 |
| Reb B G | 1000 | 13.3 | 6.9 | 2.2 | 4.0 | 2.5 | 2.0 |
| SG95 B-G | 500 | 13.3 | 5.0 | 1.8 | 4.2 | 2.4 | 1.7 |
| SG95 B-G | 1000 | 13.3 | 7.7 | 2.3 | 3.8 | 2.4 | 2.2 |
| Steviten | 500 | 6.9 | 3.8 | 1.8 | 4.4 | 2.0 | 1.4 |
| Steviten | 1000 | 6.9 | 6.6 | 2.0 | 4.4 | 2.0 | 1.7 |
| Stevioside G | 500 | 3.4 | 3.7 | 1.7 | 3.9 | 2.1 | 1.3 |
| Stevioside G | 1000 | 3.4 | 6.5 | 2.3 | 4.0 | 2.3 | 2.0 |
| Reb A G | 500 | 3.4 | 4.9 | 1.9 | 4.6 | 2.0 | 1.2 |
| Reb A G | 1000 | 3.4 | 7.6 | 1.9 | 4.4 | 2.2 | 1.7 |

The taste data indicate that all of the glycosylated samples performed better than rebaudioside A on all matrices other than SEV. At 1000 ppm, several of the samples may be considered to be at parity with rebaudioside A for sweetness, while having a smaller standard deviation in the rating. This implies that the general population of consumers would have a more consistent experience with these products relative to rebaudioside A as ingredients in consumable products such as foods and beverages, thereby providing an advantage in formulating such products. Of the compositions tested, SG95 G-B was particularly potent at 500 ppm. It would therefore stand to reason that this composition would have the lowest sweetness threshold of the compositions tested. This attribute would be of benefit in using such a composition as a flavor enhancer at low levels. The potency advantage of this composition is most evident in comparison to that of Steviten. Since a glycosylated SG95-type product, as seen with SG95 G-B, mostly consists of rebaudioside A, stevioside and their glycosylated products, it stands to reason that their taste performance would be between that of glycosylated rebaudioside A and that of glycosylated stevioside. Surprisingly, however, SG95 G-B was found to be sweeter than either of the aforementioned products. Likewise, SG95 B-G is largely made up of rebaudioside B, steviolbioside and the glycosylated products. Given that steviolbioside is reported not to be sweet relative to either rebaudioside A, rebaudioside B or stevioside and to be poor in taste quality, it was also surprising that this product (SG95 B-G) performs much like glycosylated rebaudioside B.

### Example 4

In this example, the following glycosylated steviol glycoside samples were tested:
SG95 G = Glycosylated SG 95 steviol glycoside mixture (sourced from Sweet Green Fields Corporation, containing about 60 wt% rebaudioside A and about 25 wt% stevioside), glycosylated at pH ca. 8.5, dextrin:steviol glycoside weight ratio = 2:1.
SG95 G with 4% Reb B = 96% SG95 G as above with 4% rebaudioside B (dry blended).
SG95 G with 15% SG95 B-G = 85% SG95 G with 15% SG95 B-G (as described in Example 3).
SG95 G with 20% G-B = Glycosylated SG 95 steviol glycoside mixture (sourced from Sweet Green Fields Corporation, containing about 60 wt% rebaudioside A and about 25 wt% stevioside), glycosylated at pH ca. 8.5, dextrin:steviol glycoside weight ratio = 2:1, where 20% of the material is base treated after glycosylation.

The SG95 G sample was prepared by contacting the starting steviol glycoside composition and dextrin with CGTase for up to 24 hours at 50°C. This product was then chain-shortened with amylase for 24 hours at 30°C. After an enzyme kill step (heating to 95°C for 30 mins), the product was purified by ion exchange resin, carbon filtration and adsorbent resin. The sample was then spray dried.

The SG95 G with 20%G-B sample was prepared by contacting the starting steviol glycoside composition and dextrin with CGTase for up to 18 hours at 80°C. This product was then chain shortened with amylase for 2 hours at 50°C. After an enzyme kill step (heating to 95°C for 30 mins), the residual carbohydrates were removed. 20% of the solution was then base treated at 80°C. After neutralization the material was recombined with the original solution and treated with ion exchange resin and carbon filtration. The sample was then dried using lyophilization.

Samples were tested as described in Example 3 with additional concentrations evaluated including 1500 ppm. The results obtained are summarized in Table 7.

**Table 7.**

| Composition | Conc., ppm | SEV | Liking | Off-Flavor |
|---|---|---|---|---|
| SG95 G | 1000 | 8.5 | 3.2 | 2.4 |
| SG95 G | 1500 | 8.4 | 2.5 | 3.3 |
| SG95 G with 4% Reb B | 1000 | 8.1 | 3.8 | 2.2 |
| SG95 G with 4% Reb B | 1500 | 8.7 | 3.0 | 2.9 |
| SG95 G with 15% SG95 B-G | 1000 | 7.5 | 4.4 | 1.8 |
| SG95 G with 15% SG95 B-G | 1500 | 8.7 | 3.6 | 2.8 |
| SG95 G with 20% G-B | 1000 | 7.9 | 3.8 | 2.0 |
| SG95 G with 20% G-B | 1500 | 9.0 | 3.4 | 2.3 |

As can be seen in Table 7, SG95 G (while intensely sweet) has higher off flavor and lower liking scores than the other products tested in this experiment. The inclusion of 4% rebaudioside B was found to improve the liking and off flavor scores of SG95 G. Inclusion of base treated glycosylated stevia (with base treatment before or after glycosylation), were found to further improve the taste performance of the sweetener by reduction in off flavors and/or augmentation of liking. Additionally, higher sweetening levels can be obtained likely due to the reduction of bitter off-flavor relative to the 1500 ppm solution of SG95 G.

## Claims

1. A method of improving one or more sensory characteristics of a glycosylated steviol glycoside composition, comprising contacting the glycosylated steviol glycoside composition with a basic aqueous medium or a basic resin.

2. The method of claim 1, wherein the glycosylated steviol glycoside composition is contacted with a basic aqueous medium and the basic aqueous medium has a pH of from 7.5 to 14 or 8 to 14, or from 9 to 14, or from 10 to 14.

3. The method of claim 1 or 2, wherein the contacting step is carried out at a temperature of from 20°C to 100°C.

4. The method of any of claims 1 to 3, wherein the glycosylated steviol glycoside composition is comprised of at least one of glycosylated rebaudioside A or glycosylated stevioside.

5. The method of claim 4, wherein the contacting step is carried out under conditions effective to convert at least a portion of the glycosylated rebaudioside A, if present, to glycosylated rebaudioside B and at least a portion of the glycosylated stevioside, if present, to glycosylated steviolbioside.

6. The method of any of claims 1-5, wherein the glycosylated steviol glycoside composition is obtained by a process of contacting a starting steviol glycoside composition, a glucose donor and a cyclodextrin glycosyltransferase in an aqueous medium having a pH of greater than 7.5 to not more than 10 for a time effective to produce the glycosylated steviol glycoside composition.

7. The method of any of claims 1-5, wherein the glycosylated steviol glycoside composition is obtained by a process of a) contacting a starting steviol glycoside composition with a basic aqueous medium or a basic resin to obtain a base-treated steviol glycoside composition and b) contacting the base-treated steviol glycoside composition, a glucose donor and a cyclodextrin glycosyltransferase in an aqueous medium for a time effective to produce the glycosylated steviol glycoside composition.
